(19) Europäisches Patentamt
European Patent Office
Office européen des brevets

(11) **EP 4 504 821 B1**

(12) **EUROPÄISCHE PATENTSCHRIFT**

(45) Veröffentlichungstag und Bekanntmachung des Hinweises auf die Patenterteilung:
**29.10.2025   Patentblatt 2025/44**

(21) Anmeldenummer: **22721304.8**

(22) Anmeldetag: **06.04.2022**

(51) Internationale Patentklassifikation (IPC):
**C08G 77/44** (2006.01)     **A61Q 5/00** (2006.01)

(52) Gemeinsame Patentklassifikation (CPC):
**C08G 77/44; A61K 8/898; A61Q 5/06**

(86) Internationale Anmeldenummer:
**PCT/EP2022/059157**

(87) Internationale Veröffentlichungsnummer:
**WO 2023/193903 (12.10.2023 Gazette 2023/41)**

(54) **WÄSSRIGE DISPERSIONEN VON VORVERNETZTEN ORGANOPOLYSILOXANEN**

AQUEOUS DISPERSIONS OF PRE-CROSSLINKED ORGANOPOLYSILOXANES

DISPERSIONS AQUEUSES D'ORGANOPOLYSILOXANES PRÉRÉTICULÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**12.02.2025   Patentblatt 2025/07**

(73) Patentinhaber: **Wacker Chemie AG
81671 München (DE)**

(72) Erfinder:
• **ZIRZLMEIER, Johannes
84489 Burghausen (DE)**
• **BEER, Gerhard
84489 Burghausen (DE)**
• **GRANDL, Markus
81825 München (DE)**

(56) Entgegenhaltungen:
**WO-A1-2020/239229**

**Beschreibung**

Die Erfindung betrifft wässrige Dispersionen von vorvernetzten Organopolysiloxanen, vorvernetzte Organopolysiloxane und kosmetische Zusammensetzungen enthaltend wässrige Dispersionen von vorvernetzten Organopolysiloxanen. Weiterhin betrifft die Erfindung Verfahren zur Herstellung der vorvernetzten Organopolysiloxane und deren wässriger Dispersionen. Weiterhin betrifft die Erfindung die Verwendung der kosmetischen Zusammensetzungen.

Organopolysiloxane werden in kosmetischen Zusammensetzungen, beispielsweise bei Haarpflegeprodukten, verwendet aufgrund ihrer konditionierenden Eigenschaften, wie Verbesserung von Weichheit und Glätte, Reduzierung von Kämmkräften, Glanzeigenschaften, Verbesserung von Farbeindrücken, Farbschutzeigenschaften, Verringerung der elektrostatischen Aufladungen, Schutzeigenschaften bei thermischer Belastung des Haares oder Hydrophobierung.

Eine Übersicht über ausgewählte Organopolysiloxane für Pflege keratinischer Materialien wie Haaren findet sich in M.D. Berthiaume, Society of Cosmetic Chemists (Hrsg.), Monograph, Silicones in Hair Care, 1997 und J. Sejpka, Silicone in Haarpflegeprodukten, in: SÖFW-Journal, 118. Jahrgang, Nr. 17, 1992, S. 1065-1070.

Haare sind im Alltag einer Vielzahl von äußeren Einflüssen ausgesetzt, die zur Schädigung der Haaroberfläche führen, und damit die kosmetischen Eigenschaften wie Glätte, Weichheit, Glanz und andere Parameter im Vergleich zu ungeschädigtem Haar verschlechtern. Die Schädigung der Haaroberfläche kann beispielsweise durch chemische oder mechanische Behandlung verursacht sein, durch UV-Strahlung oder durch Hitze. Einhergehend mit der Oberflächenschädigung von Haar ist die Zerstörung und teilweise Entfernung der die Cuticula bedeckenden Lipidschicht, die ursächlich für die stark hydrophobe Eigenschaft von ungeschädigtem, natürlichem Haar ist (R.A. Lodge, B. Bhusan, Wetting Properties of Human Hair by Means of Dynamic Contact Angle Measurement, Journal of Applied Polymer Science, Vol. 102, 5255-5265, 2006, Wiley). Geschädigtes Haar ist im Vergleich zu ungeschädigtem Haar deutlich hydrophiler, da nach Zerstörung der oberflächlichen Lipidschicht eine hydrophile, aminosäurebasierte Proteinmatrix als Haaroberfläche wirkt.

Modifizierte Siloxane unter Verwendung Oxalamidoesterterminierter Organopolysiloxane sind bekannt. Gemäß WO 2019/114953 A1 handelt es sich dabei um lineare Co-Polymere von Oxalamidoester-terminierten Organopolysiloxanen mit Aminoterminierten Polyethern. Diese zeigen etwa eine deutlich gesteigerte Hydrophilie gegenüber ausschließlich Aminofunktionalisierten Organopolysiloxanen. Aufgrund ihrer linearen Struktur weisen sie aber keinen elastischen Effekt auf.

In US 7501184 B2 sind Copolymere beschrieben, die durch Umsetzung von linearen Organopolysiloxanen, die mit Oxalamidoestergruppen terminiert sind, mit organischen Diaminen erhalten werden. Es werden hochviskose bis feste Copolymere erhalten, die in Klebstoffen, insbesondere als Heißschmelzkleber, eingesetzt werden. Diese hochviskosen Produkte sind nicht stabil emulgierbar und können daher nicht für die Behandlung von faserigen Substraten, wie etwa Haaren eingesetzt werden. Weiterhin weisen sie aufgrund ihrer linearen Struktur keinen elastischen Effekt auf.

In US7223385 B2, US7485289 B2, US7220408 B2 und US7504094 B2 werden kosmetische Zusammensetzungen zur Behandlung von Haaren beschrieben, die spezielle Aminosilicone enthalten sowie ein Konditionierungsmittel oder einen Verdicker. Bei den Aminosiliconen handelt es sich um endständige Alkoxy-/Hydroxygruppen aufweisende Dimethylpolysiloxane mit Aminoethylaminopropyl-Alkoxy-Siloxaneinheiten oder Aminoethylaminopropyl-Methyl-Siloxaneinheiten, die unvernetzt sind.

In WO 2020/239229 A1 werden wässrige Dispersionen von vorvernetzten Organopolysiloxanen beschrieben, die in kosmetischen Zusammensetzungen eingesetzt werden können und die vorzugsweise nach Entfernen des Wassers einen elastomeren Film bilden. Es zeigte sich, dass kosmetische Formulierungen für Haaranwendungen enthaltend die vorvernetzten Organopolysiloxane eine besondere Pflegewirkung zeigen. Trotz der Eigenschaft zur Ausbildung elastomerer Filme, beobachtet man nur eine begrenzte Wirkung bezüglich der Formgebung von Haarfasern.

Aus US5039738 A ist ein Verfahren zur Herstellung modifizierter Aminoorganosiloxane bekannt, bei dem Aminoorganosiloxane in wässrigen Emulsionen mit Dialkyloxalaten, Dialkylpyrocarbonaten oder deren Mischung umgesetzt werden. Es zeigte sich, dass sich textile Stoffe, welche mit den beschriebenen Emulsionen behandelt wurden, durch verringerte Vergilbung auszeichnen.

In WO 2015/024079 A1 werden kosmetische Zusammensetzungen beschrieben, die Aminoorganopolysiloxane, kationische Tenside und Dialkyldicarbonsäureester der Formel R'-O-CO-R-CO-O-R' beinhalten, wobei R' C8-C30-Reste beinhaltet.

Gemäß WO 2004/039930 A2 zeigen Textilien, die mit einer Zusammensetzung aus Polycarbonsäuren und Aminoorganosiloxanen behandelt wurden, eine verbesserte Kräusel- und Faltenbeständigkeit.

Eine vernetzbare Zusammensetzung aus Aminoorganopolysiloxan und einer Vernetzerkomponente, welche ein mindestens eine Carbonsäureanhydridgruppe aufweisendes Alkoxysilan oder Siloxan ist, wird in US5399652 A beschrieben.

Es bestand die Aufgabe, vorvernetze Organopolysiloxane, insbesondere wässrige Dispersionen von vorvernetzten Organopolysiloxanen, bereitzustellen, die vorzugsweise nach Entfernen des Wassers einen elastomeren Film bilden und die in kosmetischen Zusammensetzungen eingesetzt werden können. Weiterhin bestand die Aufgabe, kosmetische Zusammensetzungen zur Behandlung von keratinischen Fasern, wie Haaren, vorzugsweise zu deren Reinigung und Pflege bereitzustellen, die zur Formgebung und zum Konditionieren von keratinischen Fasern, wie Haaren, verwendet werden können, insbesondere um deren Kämmbarkeit zu erleichtern.

Die Aufgabe wird durch die Erfindung gelöst.

Gegenstand der Erfindung sind wässrige Dispersionen, vorzugsweise wässrige Emulsionen, enthaltend vorvernetzte Organopolysiloxane, die durchschnittlich mindestens eine Struktureinheit, vorzugsweise mindestens zwei Struktureinheiten, bevorzugt mindestens drei Struktureinheiten, der allgemeinen Formel

$$SiRO_{2/2}\text{-}Y\text{-}SiRO_{2/2} \qquad (I)$$

und Einheiten der Formel

$$R_2SiO_{2/2} \qquad (II)$$

enthalten, wobei

Y ein Rest der Formel

$$\text{-}R^2\text{-}[NR^3\text{-}R^4]_x\text{-}NR^3\text{-}OC\text{-}[C(Z^1)(H)]_{k1}\text{-}[C(Z^2)(H)]_{k2}\text{-}CO\text{-}NR^3$$
$$\text{-}[R^4\text{-}NR^3]_a\text{-}R^2\text{-}R_2SiO_{1/2}\text{-}[R_2SiO_{2/2}]_b\text{-}R_2SiO_{1/2}\text{-}R^2\text{-}[NR^3\text{-}R^4]_a\text{-} \qquad NR^3\text{-}OC\text{-}[C(Z^2)(H)]_{k2}\text{-} \qquad [C(Z^1)(H)]_{k1}\text{-}CO\text{-}NR^3\text{-}[R^4\text{-}NR^3]_x\text{-}R^2\text{-}$$

bedeutet,

R gleich oder verschieden sein kann und einen einwertigen SiC-gebundenen Kohlenwasserstoffrest mit 1 bis 18 Kohlenstoffatomen bedeutet, der ein oder mehrere Heteroatome aus der Gruppe N, P, S, O und Halogen enthalten kann,

$R^2$ gleich oder verschieden sein kann und einen SiC-gebundenen, zweiwertigen linearen oder verzweigten Kohlenwasserstoffrest mit 3 bis 18 Kohlenstoffatomen, vorzugsweise einen Alkylenrest mit 3 bis 10 Kohlenstoffatomen, bedeutet,

$R^3$ gleich oder verschieden sein kann und ein Wasserstoffatom, einen Alkylrest mit 1 bis 8 Kohlenstoffatomen oder einen Acylrest, wie Acetylrest, bedeutet, vorzugsweise ein Wasserstoffatom ist,

$R^4$ gleich oder verschieden sein kann und einen zweiwertigen Kohlenwasserstoffrest mit 1 bis 6 Kohlenstoffatomen, vorzugsweise einen Alkylenrest mit 1 bis 6 Kohlenstoffatomen, bedeutet,

k1 0, 1, 2 oder 3 ist, vorzugsweise 0 ist,

k2 0, 1, 2 oder 3 ist, vorzugsweise 0 ist,

x 0, 1, 2, 3 oder 4, vorzugsweise 0 oder 1 ist,

a 0, 1, 2, 3 oder 4, vorzugsweise 0 oder 1 ist,

b 0 oder eine ganze Zahl von 1 bis 500, vorzugsweise 20 bis 350, ist,

$Z^1$ -OH, H oder -NHR$^3$ bedeutet und

$Z^2$ -OH, H oder -NHR$^3$ bedeutet.

Gegenstand der Erfindung sind weiterhin vorvernetzte Organopolysiloxane, die durchschnittlich mindestens eine Struktureinheit, vorzugsweise mindestens zwei Struktureinheiten, bevorzugt mindestens drei Struktureinheiten, der allgemeinen Formel

$$SiRO_{2/2}\text{-}Y\text{-}SiRO_{2/2} \qquad (I)$$

und Einheiten der Formel

$$R_2SiO_{2/2} \qquad (II)$$

enthalten, wobei

Y ein Rest der Formel

$$\text{-}R^2\text{-}[NR^3\text{-}R^4{}_x\text{-}NR^3\text{-}OC\text{-}[C(Z^1)(H)]_{k1}\text{-}[C(Z^2)(H)]_{k2}\text{-}CO\text{-}NR^3$$
$$\text{-}[R^4\text{-}NR^3]_a\text{-}R^2\text{-}R_2SiO_{1/2}\text{-}[R_2SiO_{2/2}]_b\text{-}R_2SiO_{1/2}\text{-}R^2\text{-}[NR^3\text{-}R^4]_a\text{-} \qquad NR^3\text{-}OC\text{-}[C(Z^2)(H)]_{k2}\text{-}[C(Z^1)(H)]_{k1}\text{-}CO\text{-}NR^3\text{-}[R^4\text{-}NR^3]_x\text{-}R^2\text{-}$$

ist und

R, $R^2$, $R^3$, $R^4$, k1, k2, x, a, b, $Z^1$ und $Z^2$ die oben dafür angegebene Bedeutung haben.

Gegenstand der Erfindung ist weiterhin ein Verfahren zur Herstellung der wässrigen Dispersionen von vorvernetzten Organopolysiloxanen, indem

wässrige Dispersionen, vorzugsweise wässrige Emulsionen,
enthaltend Aminoorganopolysiloxane (1) der Formel

$$(R^1O)_dA_eR_{3-d-e}SiO(SiARO)_p(SiR_2O)_qSiR_{3-d-e}A_e(OR^1)_d \qquad (IV)$$

wobei

A ein Aminorest der allgemeinen Formel

$$-R^2-[NR^3-R^4-]_xNR^3_2$$

ist,

R gleich oder verschieden sein kann und einen einwertigen SiC-gebundenen Kohlenwasserstoffrest mit 1 bis 18 Kohlenstoffatomen bedeutet, der ein oder mehrere Heteroatome aus der Gruppe N, P, S, O, und Halogen enthalten kann,

$R^1$ gleich oder verschieden sein kann und ein Wasserstoffatom oder einen Alkylrest mit 1 bis 18 Kohlenstoffatomen bedeutet, der durch ein oder mehrere separate Sauerstoffatome unterbrochen sein kann,

$R^2$ gleich oder verschieden sein kann und einen SiC-gebundenen, zweiwertigen linearen oder verzweigten Kohlenwasserstoffrest mit 3 bis 18 Kohlenstoffatomen, vorzugsweise einen Alkylenrest mit 3 bis 10 Kohlenstoffatomen, bedeutet,

$R^3$ gleich oder verschieden sein kann und ein Wasserstoffatom, einen Alkylrest mit 1 bis 8 Kohlenstoffatomen oder einen Acylrest, wie Acetylrest, bedeutet, vorzugsweise ein Wasserstoffatom ist,

$R^4$ gleich oder verschieden sein kann und einen zweiwertigen Kohlenwasserstoffrest mit 1 bis 6 Kohlenstoffatomen, vorzugsweise einen Alkylenrest mit 1 bis 6 Kohlenstoffatomen, bedeutet,

d 0 oder 1 ist,

e 0 oder 1 ist,

p eine ganze Zahl von mindestens 1, vorzugsweise mindestens 2, bevorzugt mindestens 3, und höchstens 1000, vorzugsweise höchstens 10, ist und

q 0 oder eine ganze Zahl von 1 bis 2000, vorzugsweise 50 bis 1000, ist,

x 0, 1, 2, 3 oder 4, vorzugsweise 0 oder 1, ist,

mit reaktiven Estern (2) der Formel

$$R^5-O_2C-[C(Z^1)(H)]_{k1}-[C(Z^2)(H)]_{k2}-CO-NR^3$$

$$-[R^4-NR^3]_a-R^2-R_2SiO_{1/2}-[R_2SiO_{2/2}]_b-R_2SiO_{1/2}-R^2-[NR^3-R^4]_a- \qquad (V)$$

$$NR^3-OC-[C(Z^2)(H)]_{k2}-[C(Z^1)(H)]_{k1}-CO_2-R^5$$

wobei R, $R^2$, $R^3$ und $R^4$ die oben dafür angegebene Bedeutung haben,

$R^5$ gleich oder verschieden sein kann und einen O-gebundenen, gesättigten oder ungesättigten, linearen oder verzweigten, einwertigen Kohlenwasserstoffrest mit 1-20 Kohlenstoffatomen je Rest, der ein oder mehrere Heteroatome aus der Gruppe N, P, S, O und Halogen enthalten kann, bedeutet,

k1 0, 1, 2 oder 3 ist, vorzugsweise 0 ist,

k2 0, 1, 2 oder 3 ist, vorzugsweise 0 ist,

a 0, 1, 2, 3 oder 4, vorzugsweise 0 oder 1 ist,

b 0 oder eine ganze Zahl von 1 bis 500, vorzugsweise 20 bis 350, ist,

$Z^1$ -OH, H oder -$NHR^3$ bedeutet und

$Z^2$ -OH, H oder -$NHR^3$ bedeutet,

umgesetzt werden.

Gegenstand der Erfindung ist weiterhin ein Verfahren zur Herstellung der vorvernetzten Organopolysiloxane, dadurch gekennzeichnet, dass

Aminoorganopolysiloxane (1) der Formel

$$(R^1O)_d A_e R_{3-d-e}SiO(SiARO)_p(SiR_2O)_qSiR_{3-d-e}A_e(OR^1)_d \qquad (IV)$$

mit reaktiven Estern (2) der Formel

$$R^5\text{-}O_2C\text{-}[C(Z^1)(H)]_{k1}\text{-}[C(Z^2)(H)]_{k2}\text{-}CO\text{-}NR^3$$

$$\text{- }[R^4\text{-}NR^3]_a\text{-}R^2\text{-}R_2SiO_{1/2}\text{-}[R_2SiO_{2/2}]_b\text{-}R_2SiO_{1/2}\text{-}R^2\text{-}[NR^3\text{-}R^4]_a\text{-} \qquad (V)$$

$$NR^3\text{-}OC\text{-}[C(Z^2)(H)]_{k2}\text{-}[C(Z^1)(H)]_{k1}\text{-}CO_2\text{-}R^5$$

wobei A, R, $R^1$, $R^2$, $R^3$, $R^4$, $R^5$, d, e, p, q, k1, k2, x, a, b, $Z^1$ und $Z^2$ die oben dafür angegebene Bedeutung haben, umgesetzt werden,
und die so erhaltenen vorvernetzten Organopolysiloxane gegebenenfalls anschließend in Wasser emulgiert werden.

Vorzugsweise werden bei dem erfindungsgemäßen Verfahren als Aminoorganopolysiloxane (1) der Formel (IV) solche eingesetzt, bei denen e gleich 0 ist, die also nur seitenständige Aminoreste A enthalten und bei der Umsetzung mit den reaktiven Estern (2) Brücken gemäß Struktureinheiten der Formel (I) bilden.
Wenn e gleich 0 ist, enthalten die erfindungsgemäßen vorvernetzten Organopolysiloxane zusätzlich zu Struktureinheiten der Formel (I) und Siloxaneinheiten der Formel (II) vorzugsweise Siloxaneinheiten der Formel

$$R_{3-d}(OR^1)_dSiO_{1/2} \qquad (III)$$

wobei R, $R^1$ und d die oben dafür angegebene Bedeutung haben.
Es können aber auch Aminoorganopolysiloxane (1) der Formel (IV) eingesetzt werden, die auch endständige Aminoreste A enthalten, bei denen e also gleich 1 ist.
Es können auch Mischungen von Aminoorganopolysiloxanen (1) der Formel (IV) mit e gleich 0 und e gleich 1 eingesetzt werden. Bei der Umsetzung mit den reaktiven Estern (2) können dabei zusätzlich Brücken zwischen zwei endständigen Aminoresten (Formel (VI)) oder zwischen endständigen und seitenständigen Aminoresten (Formel (VII)) gebildet werden.
Weiterhin können zusätzlich zu den Aminoorganopolysiloxanen (1) nur endständige Aminoreste aufweisende Aminoorganopolysiloxane (1a) der Formel

$$(R^1O)_fAR_{2-f}SiO(SiR_2O)_nSiR_{2-f}A(OR^1)_f \qquad (IVa)$$

eingesetzt werden,

wobei A, R und $R^1$ die oben dafür angegebene Bedeutung haben,
f 0 oder 1 und
n eine ganze Zahl von 1 bis 1000, vorzugsweise 50 bis 1000, ist.

Die erfindungsgemäßen vorvernetzten Organopolysiloxane können daher als Brücken neben den Struktureinheiten der Formel (I) weitere Struktureinheiten der Formeln

$$SiR_2O_{1/2}\text{-}Y\text{-}SiR_2O_{1/2} \qquad (VI)$$

oder

$$SiRO_{2/2}\text{-}Y\text{-}SiR_2O_{1/2} \qquad (VII)$$

oder Mischungen aus (VI) und (VII) enthalten, wobei R und Y die oben dafür angegebene Bedeutung haben.
Bevorzugt enthalten die erfindungsgemäßen vorvernetzten Organopolysiloxane Siloxaneinheiten der Formel (III), wobei d 0 oder 1 ist, vorzugsweise 1 ist.
Die seitenständigen und ggf. endständigen Aminoreste A in den erfindungsgemäß eingesetzten Aminoorganopolysiloxanen (1) oder ggf. mitverwendeten Aminoorganopolysiloxanen (1a) können mit den reaktiven Estern (2) gegebenenfalls auch reagieren ohne Brücken zu bilden, sodass die vorvernetzten Organopolysiloxane zusätzlich Struktureinheiten der Formeln

$$SiRO_{2/2}\text{-}R^2\text{-}[NR^3\text{-}R^4]_x\text{-}NR^3\text{-}OC\text{-}[C(Z^1)(H)]_{k1}\text{-}[C(Z^2)(H)]_{k2}\text{-}CO\text{-}$$

$$NR^3\text{-}[R^4\text{-}NR^3]_a\text{-}R^2\text{-}R_2SiO_{1/2}\text{-}[R_2SiO_{2/2}]_b\text{-}R_2SiO_{1/2}\text{-}R^2\text{-}[NR^3\text{-}R^4]_a \qquad (VIII_a)$$

$$-NR^3\text{-}OC\text{-}[C(Z^2)(H)]_{k2}\text{-}[C(Z^1)(H)]_{k1}\text{-}CO_2\text{-}R^5$$

und/oder

$$SiR_2O_{1/2}\text{-}R^2\text{-}[NR^3\text{-}R^4{}_x\text{-}NR^3\text{-}OC\text{-}[C(Z^1)(H)]_{k1}\text{-}[C(Z^2)(H)]_{k2}\text{-}CO\text{-}$$

$$NR^3\text{-}[R^4\text{-}NR^3]_a\text{-}R^2\text{-}R_2SiO_{1/2}\text{-}[R_2SiO_{2/2}]_b\text{-}R_2SiO_{1/2}\text{-}R^2\text{-}[NR^3\text{-}R^4]_a \qquad (VIIIb)$$

$$-NR^3\text{-}OC\text{-}[C(Z^2)(H)]_{k2}\text{-}[C(Z^1)(H)]_{k1}\text{-}CO_2\text{-}R^5$$

enthalten können, wobei
R, $R^2$, $R^3$, $R^4$, $R^5$, k1, k2, x, a, b, $Z^1$ und $Z^2$ die oben dafür angegebene Bedeutung haben.

Im Rahmen dieser Erfindung soll Formel (IV) so verstanden werden, dass p Einheiten -(SiARO)- und q Einheiten -(SiR$_2$O)- in beliebiger Weise, beispielsweise als Block oder statistisch, im Aminorganopolysiloxanmolekül verteilt sein können.

Die erfindungsgemäß eingesetzten Aminoorganopolysiloxane (1) und ggf. mitverwendeten Aminoorganopolysiloxane (1a) können, was durch die Formeln (IV) bzw. (IVa) nicht ausgedrückt wird, auch Siloxaneinheiten ausgewählt aus der Gruppe der Formeln

$$RSiO_{3/2}, (OR^1)\,SiO_{3/2} \text{ und } SiO_{4/2} \qquad (IXa\text{-}c)$$

enthalten, wobei

R und $R^1$ die oben dafür angegebene Bedeutung haben,
so dass die erfindungsgemäßen vorvernetzten Organopolysiloxane auch Siloxaneinheiten der Formeln (IXa-c) enthalten können.

Vorzugsweise ist R ein einwertiger, gesättigter oder ungesättigter Kohlenwasserstoffrest mit 1 bis 18 Kohlenstoffatomen.

Beispiele für Kohlenwasserstoffreste R sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neoPentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Octadecylreste, wie der n-Octadecylrest; Cycloalkylreste, wie der Cyclopentyl-, Cyclohexyl-, Cycloheptylrest und Methylcyclohexylreste; Alkenylreste, wie der Vinyl-, 5-Hexenyl-, Cyclohexenyl-, 1-Propenyl-, Allyl-, 3-Butenyl- und 4-Pentenylrest; Arylreste, wie der Phenyl-, Naphthyl-, Anthryl- und Phenanthrylrest; Alkarylreste, wie o-, m-, p-Tolylreste; Xylylreste und Ethylphenylreste; und Aralkylreste, wie der Benzylrest, der α- und der β-Phenylethylrest.

Bevorzugt sind als Rest R der Methyl-, Ethyl-, Octyl- und Phenylrest, besonders bevorzugt sind der Methyl- und Ethylrest.

Beispiele für substituierte Reste R sind Halogenalkylreste, wie der 3,3,3-Trifluor-n-propylrest, der 2,2,2,2',2',2'-Hexafluorisopropylrest, der Heptafluorisopropylrest und Halogenarylreste, wie der o-, m- und p-Chlorphenylrest.

Weitere Beispiele für substituierte Reste R sind Polyalkylenoxygruppen, wie Polyethylenoxy-, Polypropylenoxy- oder Polyethylenoxy/Polypropylenoxy-Gruppen.

Beispiele für Rest $R^1$ sind die vorstehend bei R aufgeführten Alkylreste sowie der Methoxyethyl-, der Ethoxyethylrest und der Hexoxyethylrest, wobei es sich bei dem Rest $R^1$ bevorzugt um Wasserstoff, den Methyl- oder den Ethylrest handelt.

Vorzugsweise ist $R^5$ ein $C_{1-20}$-Kohlenwasserstoffrest, der durch ein oder mehrere Sauerstoffatome unterbrochen sein kann. Beispiele für Reste R gelten auch für Reste $R^5$.

Bevorzugt ist $R^5$ ein $C_{1-4}$-Alkylrest, wie Methyl- oder Ethylrest.

Beispiele für Reste A sind:

-(CH_2)_3NH_2

-(CH_2)_3-NH-(CH_2)_2-NH_2

-CH_2CH(CH_3)CH_2-NH-(CH_2)_2-NH_2

-(CH_2)_3-NH(Cyclohexyl)

-(CH_2)_3-NHCH_3

-(CH_2)_3-N(CH_3)_2

-(CH_2)_3-NHCH_2CH_3

-(CH_2)_3-N(CH_2CH_3)_2

-(CH_2)_4-NH_2

-CH_2CH(CH_3)CH_2-NH_2

-(CH_2)_3-NH-(CH_2)_2-NHCH_3

-(CH_2)_3-NH-(CH_2)_2-N(CH_3)_2

-(CH_2)_3-NH-(CH_2)_2-NHCH_2CH_3

-(CH_2)_3-NH-(CH_2)_2-N(CH_2CH_3)_2

-(CH_2)_3[-NH-CH_2CH_2]_2-NH_2

und deren teil- oder vollacetylierte Formen, wie

-(CH_2)_3-NH(Acetyl)

-(CH_2)_3-NH-(CH_2)_2-NH(Acetyl) und

-(CH_2)_3-N(Acetyl)-(CH_2)_2-NH(Acetyl).

Bevorzugte Beispiele für Reste A sind:

-(CH_2)_3NH_2

-(CH_2)_3-NH-(CH_2)_2-NH_2

-CH_2CH(CH_3)CH_2-NH-(CH_2)_2-NH_2

-(CH_2)_3-NHCH_3

Bevorzugt ist A ein Aminorest der Formel

-R^2-[NH-CH_2CH_2-]_xNH_2

wobei

x 0 oder 1 ist und
R^2 ein Rest der Formel -(CH_2)_3- oder -CH_2-CH(CH_3)-CH_2- ist.

Besonders bevorzugte Beispiele für Rest A sind

$$-(CH_2)_3NH_2$$

$$-(CH_2)_3-NH-(CH_2)_2-NH_2 \text{ und}$$

$$-CH_2CH(CH_3)CH_2-NH-(CH_2)_2-NH_2.$$

Weitere Beispiele für Aminoorganopolysiloxane (1) sind handelsübliche funktionalisierte Siloxane, wie Aminöle, z.B. Aminöle mit 3-(2-Aminoethyl)aminopropylfunktionen, sowie Glykolöle, Phenyl- oder Phenylmethylöle, die Aminogruppen enthalten.

Bei der Herstellung der erfindungsgemäßen Dispersionen kann eine Art von Aminoorganopolysiloxan (1) oder verschiedene Arten von Aminoorganopolysiloxan (1) eingesetzt werden.

Die bei der Herstellung der erfindungsgemäßen Dispersionen eingesetzten Aminoorganopolysiloxane (1) weisen vorzugsweise Viskositäten von 1 mPa.s bis 50.000.000 mPa.s bei 25°C, bevorzugt 50 mPa.s bis 10.000.000 mPa.s bei 25°C und besonders bevorzugt 100 mPa.s bis 500.000 mPa.s bei 25°C auf.

Die bei der Herstellung der erfindungsgemäßen Dispersionen eingesetzten Aminoorganopolysiloxane (1) können beispielsweise, wie in US 7,129,369 B2 beschrieben, hergestellt werden.

Die erfindungsgemäßen Dispersionen enthalten vorzugsweise erfindungsgemäße vorvernetzte Organopolysiloxane,

Emulgatoren (3) und
Wasser (4).

Bei der Herstellung der erfindungsgemäßen Dispersionen von vorvernetzten Organopolysiloxanen können gegebenenfalls weitere Stoffe, die an der Umsetzung nicht direkt teilnehmen, eingesetzt werden.

Die erfindungsgemäßen Dispersionen bilden beim Eintrocknen - ohne Katalysatorzugabe oder Veränderung des pH-Wertes - ein Siliconnetzwerk, vorzugsweise ein elastisches Siliconnetzwerk aus. Vorzugsweise bilden die erfindungsgemäßen Dispersionen nach dem Entfernen des Wassers elastomere Filme.

Bei dem erfindungsgemäßen Verfahren, der erfindungsgemäßen Umsetzung von Aminoorganopolysiloxanen (1) und ggf. (1a) mit reaktiven Estern (2), werden vorzugsweise keine metallhaltigen Katalysatoren mitverwendet.

Die erfindungsgemäßen Dispersionen enthalten daher vorzugsweise keine Katalysatoren.

Bei der Herstellung der erfindungsgemäßen vorvernetzten Organopolysiloxane werden Aminoorganopolysiloxane (1) und reaktive Ester (2) eingesetzt, und diese Komponenten reagieren vorzugsweise bei Raumtemperatur miteinander. Zur Unterstützung dieser Reaktion werden keine metallhaltigen, zusätzlichen Katalysatoren benötigt, d.h. es werden vorzugsweise keine Übergangsmetalle der VIII. Nebengruppe des Periodensystems und deren Verbindungen und keine Metalle der III., IV. und V. Hauptgruppe des Periodensystems und deren Verbindungen verwendet, wobei die Elemente C, Si, N, und P in dieser Definition nicht als Metalle gelten.

Die Reaktion verläuft ferner vorzugsweise im neutralen Bereich, d.h. im pH-Bereich von ca. 4 bis 8, der sich durch die Komponenten selbst ergibt. Durch die hohe Reaktivität ist ferner eine gezielt geführte chemische Umsetzung nicht nötig und vorzugsweise auch kein Erwärmen.

Bei der Herstellung der erfindungsgemäßen Dispersionen kann eine Art von Ester (2) als Vernetzer oder verschiedene Arten von Estern (2) als Vernetzer eingesetzt werden.

Vorzugsweise werden als Ester (2) Oxalamidoester-terminierte Organopolysiloxane der Formel

$$R^5-O_2C-CO-NH-R^2-R_2SiO_{1/2}-[R_2SiO_{2/2}]_b-R_2SiO_{1/2}-R^2-NH-OC-CO_2-R^5$$

eingesetzt, wobei

R die oben dafür angegebene Bedeutung hat,
$R^2$ ein Rest der Formel $-(CH_2)_3-$ oder $-CH_2-CH(CH_3)-CH_2-$ ist,
$R^5$ die oben dafür angegebene Bedeutung hat, bevorzugt ein $C_{1-4}$-Alkylrest, insbesondere ein Methyl- oder Ethylrest ist und
b gleich 0 oder eine ganze Zahl von 1 bis 500, vorzugsweise 20 bis 350, ist.

Bevorzugt ist Y daher ein Rest der Formel

$$-R^2-[NH-CH_2CH_2]_x-NH-OC-CO-NH-R^2 \qquad -R_2SiO_{1/2}-[R_2SiO_{2/2}]_b-R_2SiO_{1/2}- \qquad R^2-NH-OC-CO-$$

NH-[CH$_2$CH$_2$-NH]$_x$-R$^2$-

wobei x 0 oder 1 ist,

b und R die oben dafür angegebene Bedeutung haben und
R$^2$ ein Rest der Formel -(CH$_2$)$_3$- oder -CH$_2$-CH(CH$_3$)-CH$_2$- ist.

Die vorvernetzten Organopolysiloxane können je nach Einsatz von Vernetzer (2) oder linearem, verzweigtem oder harzartigem Aminoorganopolysiloxan (1) verzweigte oder sogar hochverzweigte bzw. stark vernetzte Strukturen mit linearen Anteilen haben.

Bei dem erfindungsgemäßen Verfahren werden Aminoorganopolysiloxane und Ester in Art und Menge so gewählt werden, dass die Organopolysiloxane in den erhaltenen Dispersionen vorvernetzt sind.

Vorzugsweise werden als Aminoorganopolysiloxane (1) solche eingesetzt, die durchschnittlich mindestens eine primäre Aminofunktion in den seitenständigen Aminoresten A enthalten, so dass erfindungsgemäße vorvernetzte Organopolysiloxane erhalten werden. Es können zusätzlich auch Aminoorganopolysiloxane (1a) eingesetzt werden, die nur endständige Aminoreste A mit primären Aminfunktionen enthalten.

Wenn Aminoorganopolysiloxane (1a) eingesetzt werden, werden sie vorzugsweise in Mengen von 10 bis 300 Gew.-teilen, je 100 Gew.- teile Aminoorganopolysiloxane (1) eingesetzt.

Ferner werden auch bei Einsatz von Gemischen aus Aminoorganopolysiloxanen (1) und unfunktionellen Organopolysiloxanen (1b), erfindungsgemäße vorvernetzte Organopolysiloxane erhalten. Unfunktionelle Organopolysiloxane (1b) sind dabei Organopolysiloxane ohne Aminoreste A, vorzugsweise lineare Organopolysiloxane der allge-meinen Formel

$$R^7{}_uR^6{}_{3-u}SiO[R^6{}_2SiO]_vSiR^6{}_{3-u}R^7{}_u \qquad (X)$$

wobei R$^6$ die Bedeutung von R hat und

R$^7$ die Bedeutung von R$^6$ hat oder eine HO-Gruppe ist,
u 0 oder 1 und
v 0 oder eine ganze Zahl von 1 bis 2000 ist.

Vorzugsweise ist R$^6$ ein C$_{1-18}$-Kohlenwasserstoffrest, bevorzugt ein C$_{1-18}$-Alkylrest.

Beispiele für unfunktionelle Organopolysiloxane (1b) sind Dialkylpolysiloxane, bevorzugt Dimethylpolysiloxane.

Wenn unfunktionelle Organopolysiloxane (1b) eingesetzt werden, werden sie vorzugsweise in Mengen von 100 bis 800 Gew.-teilen je 100 Gew.-teile Aminoorganopolysiloxane (1) eingesetzt.

Der Vernetzungsgrad hängt dabei vom eingesetzten Verhältnis der Äquivalente -OR$^5$ des reaktiven Esters (2) zum Aminorest A im Aminoorganopolysiloxan (1) ab.

Zur Herstellung der erfindungsgemäßen Dispersionen aus Aminoorganopolysiloxan (1) und reaktivem Ester (2) wird der Ester (2) dabei vorzugsweise in Mengen von 0,1 bis 10 Äquivalente -OR$^5$, bevorzugt 0,2 bis 5 Äquivalente -OR$^5$, besonders bevorzugt 0,3 bis 3 Äquivalente -OR$^5$, je Äquivalent Aminorest A im Aminoorganopolysiloxan (1) eingesetzt.

Die Herstellung der erfindungsgemäßen Dispersionen von vorvernetzten Organopolysiloxanen erfolgt durch intensives Mischen von Aminoorganopolysiloxanen (1) mit Estern (2), Emulgatoren (3) und Wasser (4) miteinander. Die Herstellung kann diskontinuierlich oder kontinuierlich erfolgen.

Die Art der Mischung der Komponenten, die zur Herstellung der erfindungsgemäßen Dispersionen gebraucht werden, ist nicht sehr kritisch und kann in verschiedener Reihenfolge ausgeübt werden.

Es können z.B. die Komponenten (1) und (2) miteinander vorgemischt werden, daraufhin der (oder die) Emulgator(en) zugefügt und danach Wasser (4) eingearbeitet werden. Es ist auch möglich, die Komponenten (1) bis (4) der Reihe nach in die Emulgierapparatur zu dosieren. In besonderen Fällen kann es z.B. aufgrund der Viskosität oder Reaktivität der eingesetzten Aminoorganopolysiloxane (1) und ggf. (1a) und ggf. unfunktionellen Organopolysiloxane (1b) vorteilhaft sein, Ester (2) mit einem Aminoorganopolysiloxan zu mischen und danach ein anderes Aminoorganopolysiloxan oder unfunktionelles Organopolysiloxan einzuarbeiten, oder umgekehrt, je nachdem, wie sich günstigere rheologische Eigenschaften für die Verarbeitung der Komponenten ergeben.

Des Weiteren ist es auch möglich, den Ester (2) als Vernetzer in die fertige Emulsion von Aminoorganopolysiloxanen (1) zu geben, um so die gewünschte Reaktion und Vernetzung des Aminoorganopolysiloxans in der Emulsion zu erreichen. Um VOCfreie Produkte, also Produkte ohne flüchtige organische Verbindungen, zu erhalten kann das Nebenprodukt Alkohol R$^5$OH (wobei R$^5$ die oben dafür angegebene Bedeutung hat) durch geeignete bekannte Maßnahmen wie Destillation, Membranverfahren oder andere Trennverfahren teilweise oder vollständig entfernt werden.

Bei der Herstellung der erfindungsgemäßen Dispersionen wird Wasser (4), in Mengen von vorzugsweise 1 bis 99 Gew.-%, besonders bevorzugt 25 bis 95 Gew.-%, jeweils bezogen auf das Gesamtgewicht aller Inhaltsstoffe der Dispersion eingesetzt.

Die erfindungsgemäße wässrige Dispersion von vorvernetzten Organopolysiloxanen wird als Öl in Wasser System eingesetzt.

Als Emulgatoren (3) zur Herstellung der wässrigen Dispersionen von vorvernetzten Organopolysiloxanen können alle bisher bekannten, anionische, nichtionische, kationische oder amphotere Emulgatoren sowohl einzeln als auch als Mischungen verschiedener Emulgatoren verwendet werden, mit denen auch bisher wässrige Dispersionen, insbesondere wässrige Emulsionen von Organopolysiloxanen hergestellt werden konnten.

Beispiele für anionische Emulgatoren sind:

1. Alkylsulfate, besonders solche mit einer Kettenlänge von 8 bis 18 C-Atomen, Alkyl- und Alkarylethersulfate mit 8 bis 18 C-Atomen im hydrophoben Rest und 1 bis 40 Ethylenoxid (EO)- bzw. Propylenoxid(PO)einheiten.

2. Sulfonate, besonders Alkylsulfonate mit 8 bis 18 C-Atomen, Alkylarylsulfonate mit 8 bis 18 C-Atomen, Tauride, Ester und Halbester der Sulfobernsteinsäure mit einwertigen Alkoholen oder Alkylphenolen mit 4 bis 15 C-Atomen; gegebenenfalls können diese Alkohole oder Alkylphenole auch mit 1 bis 40 EO-Einheiten ethoxyliert sein.

3. Alkali- und Ammoniumsalze von Carbonsäuren mit 8 bis 20 C-Atomen im Alkyl-, Aryl-, Alkaryl- oder Aralkylrest.

4. Phosphorsäureteilester und deren Alkali- und Ammoniumsalze, besonders Alkyl- und Alkarylphosphate mit 8 bis 20 C-Atomen im organischen Rest, Alkylether- bzw. Alkaryletherphosphate mit 8 bis 20 C-Atomen im Alkyl- bzw. Alkarylrest und 1 bis 40 EO-Einheiten.

Beispiele für nichtionische Emulgatoren sind:

5. Polyvinylalkohol, der noch 5 bis 50%, vorzugsweise 8 bis 20% Vinylacetateinheiten aufweist, mit einem Polymerisationsgrad von 500 bis 3000.

6. Alkylpolyglycolether, vorzugsweise solche mit 3 bis 40 EO-Einheiten und Alkylresten von 8 bis 20 C-Atomen.

7. Alkylarylpolyglycolether, vorzugsweise solche mit 5 bis 40 EO-Einheiten und 8 bis 20 C-Atomen in den Alkyl- und Arylresten.

8. Ethylenoxid/Propylenoxid(EO/PO)-Blockcopolymere, vorzugsweise solche mit 8 bis 40 EO- bzw. PO-Einheiten.

9. Additionsprodukte von Alkylaminen mit Alkylresten von 8 bis 22 C-Atomen mit Ethylenoxid oder Propylenoxid.

10. Fettsäuren mit 6 bis 24 C-Atomen.

11. Alkylpolyglykoside der allgemeinen Formel R''-O-$Z_O$, worin R'' einen linearen oder verzweigten, gesättigten oder ungesättigten Alkylrest mit im Mittel 8 - 24 C-Atomen und $Z_O$ einen Oligoglykosidrest mit im Mittel o = 1 - 10 Hexose- oder Pentoseeinheiten oder Gemischen davon bedeuten.

12. Naturstoffe und deren Derivate, wie Lecithin, Lanolin, Saponine, Cellulose; Cellulosealkylether und Carboxyalkylcellulosen, deren Alkylgruppen jeweils bis zu 4 Kohlenstoffatome besitzen.

13. Polare Gruppen, enthaltend insbesondere die Elemente O, N, C, S, P, Si, enthaltende lineare Organo(poly) siloxane, insbesondere solche mit Alkoxygruppen mit bis zu 24 C-Atomen und/oder bis zu 40 EO- und/oder PO-Gruppen.

Beispiele für kationische Emulgatoren sind:

14. Salze von primären, sekundären und tertiären Fettaminen mit 8 bis 24 C-Atomen mit Essigsäure, Schwefelsäure, Salzsäure und Phosphorsäuren.

15. Quarternäre Alkyl- und Alkylbenzolammoniumsalze, insbesondere solche, deren Alkylgruppen 6 bis 24 C-Atome besitzen, insbesondere die Halogenide, Sulfate, Phosphate und Acetate.

16. Alkylpyridinium-, Alkylimidazolinium- und Alkyloxazoliniumsalze, insbesondere solche, deren Alkylkette bis zu 18 C-Atome besitzt, speziell die Halogenide, Sulfate, Phosphate und Acetate.

Als amphotere Emulgatoren eignen sich besonders:

17. Langkettig substituierte Aminosäuren, wie N-Alkyl-di-(aminoethyl-)glycin oder N-Alkyl-2-aminopropionsäuresalze.

18. Betaine, wie N-(3-Acylamidopropyl)-N,N-dimethylammonium-salze mit einem $C_8$-$C_{18}$-Acylrest und Alkyl-imidazolium-Betaine oder quaternisierte Alkyl oder substituierte Alkylderivate des N,N-Dimethyl-Glycins.

Bevorzugt als Emulgatoren zur Herstellung für wässrige Dispersionen vorvernetzter Organopolysiloxane sind nichtionische Emulgatoren, insbesondere die vorstehend unter 6. aufgeführten Alkylpolyglycolether.

Der Bestandteil (3) kann aus einem der o.g. Emulgatoren oder aus einem Gemisch zweier oder mehrerer o.g. Emulgatoren bestehen, er kann in reiner Form oder als Lösungen eines oder mehrerer Emulgatoren in Wasser oder organischen Lösungsmitteln eingesetzt werden.

Bei der Herstellung der erfindungsgemäßen Dispersionen werden die Emulgatoren (3) in Mengen von vorzugsweise 0,1 bis 60 Gew.-%, besonders bevorzugt 0,5 bis 30 Gew.-%, jeweils bezogen auf das Gesamtgewicht an Aminoorganopolysiloxanen (1) und Ester (2), eingesetzt.

Falls das Aminoorganopolysiloxan (1) oder der Ester (2) bzw. das entstehende vorvernetzte Organopolysiloxan selbst als Emulgator wirkt, kann auf den Zusatz von separatem Emulgator (3) verzichtet werden.

Der Emulgiervorgang zur Herstellung der Dispersion wird vorzugsweise bei Temperaturen unter 120°C, bevorzugt bei 5°C bis 100°C, besonders bevorzugt bei 10°C bis 80°C durchgeführt. Die Temperaturerhöhung kommt vorzugsweise durch den Eintrag mechanischer Scherenergie, die für den Emulgierprozess benötigt wird, zustande. Die Temperaturerhöhung wird nicht zur Beschleunigung eines chemischen Prozesses benötigt. Weiterhin erfolgt die Herstellung der Dispersionen vorzugsweise beim Druck der umgebenden Atmosphäre, sie kann aber auch bei höheren oder niedrigeren Drücken durchgeführt werden.

Die Umsetzung von Aminoorganopolysiloxanen (1) mit Estern (2) bei der Herstellung der Dispersionen läuft vorzugsweise in wenigen Minuten bis mehreren Tagen ab.

Die bei der Herstellung der Dispersionen als Kondensationsnebenprodukte anfallenden Alkohole können im Produkt verbleiben oder auch entfernt werden, beispielsweise durch Destillation unter Vakuum, Membranverfahren, oder durch Extraktion.

Die mithilfe von Laserbeugung (mit dem Mastersizer 3000 Particle Size Analyzer der Firma Malvern Panalytical) in den erfindungsgemäßen Dispersionen gemessene mittlere Teilchengröße liegt vorzugsweise im Bereich 0,001 bis 50 µm, bevorzugt bei 0,005 bis 20 µm, besonders bevorzugt im Bereich 0,01 bis 10 µm.

Die pH-Werte können von 1 bis 14 variieren, bevorzugt 3 bis 9, besonders bevorzugt 4 bis 8.

Gegenstand der Erfindung sind kosmetische Zusammensetzungen enthaltend erfindungsgemäße wässrige Dispersionen, vorzugsweise wässrige Emulsionen, von vorvernetzten Organopolysiloxanen.

Die erfindungsgemäße kosmetische Zusammensetzung enthält wässrige Dispersionen von vorvernetzten Organopolysiloxanen vorzugsweise in Mengen von 0,2 bis 65 Gew.-%, bevorzugt von 0,5 bis 50 Gew.-%, jeweils bezogen auf das Gesamtgewicht der kosmetischen Zusammensetzung.

Die erfindungsgemäßen kosmetischen Zusammensetzungen enthalten vorzugsweise als kosmetisch akzeptables Medium Wasser.

Die erfindungsgemäße kosmetische Zusammensetzung enthält vorzugsweise ein Konditionierungsmittel. Als Konditionierungsmittel werden analog zu K. Krummel, Stephane Chiron, J. Jachowicz, Chapter 14, in: "The Chemistry and Manufacture of Cosmetics", Volume II, Formulating, Third Edition by Mitchell L. Schlossmann, 2000, S. 359-396, kosmetische Inhaltsstoffe bezeichnet, welche die Haaroberfläche modifizieren und die Beschaffenheit des Haares beeinflussen. Kosmetische Zusammensetzungen enthaltend Konditionierungsmittel werden eingesetzt zur Modifizierung oder Verbesserung der Weichheit der Haare, besseren Entwirrbarkeit, Verringerung der Nass- und Trockenkämmkraft, Pflege der Haare, Vermeidung elektrostatischer Aufladung, leichterer Gleitwirkung durch das Haar und entlang der Haaroberfläche, Verbesserung des Haarglanzes, Erhalt der Farbechtheit von Haaren, Verringerung des Haarbruchs, Erhalt der Haarform und weiteren kosmetischen Eigenschaften, die mit natürlichem und gesundem Haar in Verbindung gebracht werden.

Die erfindungsgemäße kosmetische Zusammensetzung verbessert einen oder mehrere der oben genannten Effekte, insbesondere die Kämmbarkeit und die Deponierung des Silicons auf den Haaren.

Beispiele für Konditionierungsmittel und deren INCI-Namen sind beschrieben im "International Cosmetic Ingredient Dictionary & Handbook" des Personal Care Product Council (Hrsg.).

Als Referenz kann das World Wide Web basierte "wINCI Web Based International Cosmetic Ingredient Dictionary & Handbook (http://online.personalcarecouncil.org/jsp/Home.jsp) oder das International Cosmetic Ingredient Dictionary & Handbook, 13th Edition, The Personal Care Products Council (formerly: The Cosmetic, Toiletry, and Fragrance Association (CTFA)), 2010, Verwendung finden.

Als Konditionierungsmittel werden vorzugsweise solche ausgewählt aus der Gruppe von kationischen Polymeren,

kationischen Tensiden,
nicht polymer vorliegenden quaternären Ammoniumverbindungen,
Organopolysiloxanen und Organopolysiloxancopolymeren, die von den vorvernetzten Organopolysiloxanen enthaltend Struktureinheiten der Formel (I) verschieden sind,
Fettsäureestern und Fettsäurealkoholen,
natürlichen oder synthetischen Ölen und Wachsen und
Panthenol, Lipiden, Proteinen und hydrolysierten Proteinen, sowie deren Mischungen
eingesetzt.

Bevorzugte Beispiele für Konditionierungsmittel sind kationische Polymere. Darunter verstanden werden Polymere, die seitenständig oder endständig kationische Gruppen tragen oder seitenständig oder endständig Gruppen, die durch Ionisierung in eine kationische Gruppe überführt werden können.

Vorzugsweise werden kationische Polymere verwendet, die eine quaternäre Ammoniumgruppe aufweisen.

Beispiele für vorzugsweise eingesetzte kationische Polymere sind im International Cosmetic Ingredient Dictionary & Handbook unter der Bezeichnung Polyquaternium veröffentlicht, wobei jedes Polymer mit einem individuellen Zahlenkürzel identifiziert wird, z.B. Polyquaternium-1.

Weitere Beispiele für kationische Polymere sind quaternäre Ammoniumgruppen aufweisende Derivate von modifizierten Polysacchariden, z.B. Polymere mit den INCI-Namen Cassia Hydroxypropyltrimonium Chloride, Derivate von modifizierter Cellulose und/oder Stärke, z.B. ein quaternäres Ammoniumderivat eines mit Propylenglycolether modifizierten Cyamopsis Tetragonoloba (Guar) Gums mit dem INCI-Namen Guar Hydroxypropyltrimonium Chloride, oder polymere quaternäre Ammoniumsalze des Reaktionsprodukts von Hydroxyethylcellulose mit einem Trimethylammonium-substituierten Epoxid, wie Cellulose, 2-Hydroxyethyl 2-(2-Hydroxy-3-(Trimethylammonium)Propoxy)Ethyl 2-Hydroxy-3-(Trimethylammonio)Propyl Ether Chlorid, wie Cellulose, 2-Hydroxyethyl 2-Hydroxy-3-(Trimethylammonium) Propyl Ether, Chlorid, wie Cellulose, 2-Hydroxyethyl 2-Hydroxy-3-(Trimethylammonium)Propyl Ether chloride, wie Cellulose, 2-[2-Hydroxy-3-(Trimethylammonium)Propoxy]Ethyl Ether, Chlorid, wie Cellulose, 2-[2-Hydroxy-3-Trimethylammonium)propoxy] Ethyl ether chloride mit dem INCI-Namen Polyquaternium-10.

Weitere Beispiele für kationische Polymere sind quaternäre Ammoniumgruppen aufweisende Acrylsäurepolymerderivate, Acrylsäurecopolymerderivate, Methacrylsäurederivate und Methacrylsäurecopolymerderivate, z.B. Polymere mit dem INCI-Namen Polyquaternium-37.

Weitere Beispiele für kationische Polymere sind quaternäre Ammoniumgruppen aufweisende Copolymere aus Dimethyldiallylammoniumchlorid und Acrylsäure, z.B. Polymere mit dem INCI-Namen Polyquaternium-22.

Weitere Beispiele für kationische Polymere sind quaternäre Ammoniumgruppen aufweisende Copolymere aus Derivaten des Vinylpyrrolidon, Viylimidazol und Vinylimidazolin und Methacrylsäure, z.B. Polymere mit dem INCI-Namen Polyquaternium-86.

Weitere Beispiele für kationische Polymere sind quaternäre Ammoniumgruppen aufweisende Copolymere aus Acrylamid und Dimethyl-diallyl-ammoniumchlorid, z.B. Polymere mit dem INCI-Namen Polyquaternium-7.

Weitere Beispiele für kationische Polymere sind quaternäre Ammoniumgruppen aufweisende Copolymere aus dem Reaktionsprodukt von Diethylsulfat mit Vinylpyrrolidon und Dimethylaminoethylmethacrylat, z.B. Polymere mit dem INCI-Namen Polyquaternium-11.

Wenn kationische Polymere eingesetzt werden, enthält die erfindungsgemäße kosmetische Zusammensetzung kationische Polymere vorzugsweise in Mengen von 0,01 bis 5 Gew.-%, bevorzugt von 0,05 bis 4 Gew.-%, insbesondere bevorzugt 0,10 bis 3 Gew.-%, jeweils bezogen auf das Gesamtgewicht der kosmetischen Zusammensetzung.

Weitere bevorzugte Beispiele für Konditionierungsmittel sind kationische Tenside. Beispiele für vorzugsweise eingesetzte kationische Tenside entsprechen den in Punkt 14. bis 16. unter Beispiele für kationische Emulgatoren aufgeführten Materialien. Beispiele sind Cetyltrimethylammoniumsalze oder Behenyltrimethylammoniumsalze. Als anionisches Gegenion können beispielsweise Chlorid, Bromid, Methosulfat vorliegen. INCI-Namen von vorzugsweise eingesetzten kationischen Tensiden sind beispielsweise Cetrimonium Chloride, Cetrimonium Methosulfate, Behentrimonium Chloride, Behentrimonium Methosulfate, Steartrimonium Bromide.

Wenn kationische Tenside eingesetzt werden, enthält die erfindungsgemäße kosmetische Zusammensetzung kationische Tenside vorzugsweise in Mengen von 0,1 bis 7 Gew.-%, bevorzugt von 0,15 bis 6 Gew.-%, insbesondere bevorzugt 0,2 bis 5 Gew.-%, jeweils bezogen auf das Gesamtgewicht der kosmetischen Zusammensetzung.

Weitere Beispiele für Konditionierungsmittel sind nicht polymer vorliegende quaternäre Ammoniumverbindungen. Darunter verstanden werden nicht polymere Ammoniumverbindungen, die kationisch vorliegen oder durch Ionisierung in eine kationische Gruppe überführt werden können.

Beispiele für vorzugsweise eingesetzte nicht polymer vorliegende quaternäre Ammoniumverbindungen sind Dimethyl Dioctadecyl Ammonium Chloride mit dem INCI-Namen Distearyldimonium Chloride, N-[3-(Dimethylamino) propyl]octadecanamid mit dem INCI-Namen Stearamidopropyl Dimethylamine oder Verbindungen mit dem INCI-Namen Dicocoylethyl Hydroxyethylmonium Methosulfate oder Quaternium-87.

Weitere bevorzugte Beispiele für Konditionierungsmittel sind Organopolysiloxane und Organopolysiloxancopolymere, die von den in den wässrigen Dispersionen vorliegenden vorvernetzten Organopolysiloxanen mit Struktureinheiten der Formel (I) verschieden sind. Die Organopolysiloxane können in Form eines Öls, Wachses, Gummis oder Harzes vorliegen oder in Form einer Emulsion.

Beispiele für solche von den vorvernetzten Organopolysiloxanen mit Struktureinheiten der Formel (I) verschiedenen Organopolysiloxane sind:

Cyclische Organopolysiloxane der Formel

$$[R^*_2SiO]_{x'}$$

wobei x' eine ganze Zahl von 4 bis 8 ist,
lineare Organopolysiloxane der allgemeinen Formel $R^*_3SiO[R^*_2SiO]_ySiR^*_3$ oder

$$HOSiR^*_2O[R^*_2SiO]_ySiR^*_2OH \,,$$

wobei y 0 oder eine ganze Zahl von 1 bis 2000 ist,
und harzartige Organopolysiloxane der allgemeinen Formel

$$R^*_tSiO_{(4-t)/2}$$

wobei R* jeweils die oben dafür angegebene Bedeutung von R, R$^1$ oder A, vorzugsweise von R und R$^1$, hat, und t 0, 1, 2 oder 3 ist,
so dass das Organopolysiloxanharz aus M, D, T und / oder Q-Einheiten aufgebaut ist, wobei die Kombination vorwiegend oder ausschließlich aus D und T-Einheiten ebenso bevorzugt sind, wie die Kombination vorwiegend oder ausschließlich aus M und Q-Einheiten, wobei im Falle der vorwiegend oder ausschließlich aus D und T-Einheiten aufgebauten Harze T-Einheiten bevorzugt in einem Molverhältnis von T/[M+D+T+Q] von 0,45 bis 1, besonders bevorzugt von 0,55 bis 1,0 vorliegen und die Anzahl der M und Q-Einheiten in beiden Fällen vorzugsweise Null ist und in dem Falle der vorwiegend oder ausschließlich aus M und Q-Einheiten aufgebauten Organopolysiloxanharze Q-Einheiten bevorzugt in einem Molverhältnis von Q/[M+D+T+Q] von 0,25 bis 0,9, besonders bevorzugt von 0,35 bis 0,7 vorliegen und die Anzahl der D und T-Einheiten in beiden Fällen vorzugsweise Null ist.

Beispiele für Organopolysiloxane, vorliegend in Form eines Öls sind Polydimethylsiloxane mit der Viskosität 0,65 bis 2 000 000 mPas (25°C) und den INCI-Bezeichnungen Disiloxan und Dimethicone.

Weitere Beispiele für Organopolysiloxane, vorliegend in Form eines Öls oder Wachses sind funktionalisierte Organopolysiloxane, beispielsweise Polyalkysiloxane, wobei mindestens ein Alkylrest sich unterscheidet von Methyl, beispielsweise Organopolysiloxane mit dem INCI-Namen Stearyl Dimethicone, Cetyl Dimethicone oder C26-28 Alkyl Dimethicone, oder beispielsweise Polyarylsiloxane und Polyarylalkylsiloxane, beispielsweise Organopolysiloxane mit dem INCI-Namen Phenyl Trimethicone, Trimethylsiloxyphenyl Dimethicone oder Dimethylphenyl Dimethicone, oder beispielsweise Organopolysiloxane mit einem organofunktionellen Rest wie einem Aminopropyl-, Aminopropyl-aminoethyl-, Aminopropylaminoisobutylrest, beispielsweise Organopolysiloxane mit dem INCI-Namen Amodimethicone, oder beispielsweise Organopolysiloxane mit einem Polyethylenglycol- oder Polyalkylenglycolrest, beispielsweise Organopolysiloxane mit dem INCI-Namen PEG-12 Dimethicone, PEG/PPG-25,25-Dimethicone oder Cetyl PEG/PPG-15/15 Butyl Ether Dimethicone.

Weitere Beispiele für Organopolysiloxane sind Siliconharze mit den INCI-Namen Trimethylsiloxysilicate oder Polymethylsilsesquioxane.

Wenn solche Organopolysiloxane oder Organopolysiloxancopolymere eingesetzt werden, enthält die erfindungsgemäße kosmetische Zusammensetzung Organopolysiloxane und Organopolysiloxancopolymere, die von den in den wässrigen Dispersionen vorliegenden vorvernetzten Organopolysiloxanen mit Struktureinheiten der Formel (I) verschieden sind, vorzugsweise in Mengen von 0,1 bis 40 Gew.-%, bevorzugt von 0,2 bis 30 Gew.-%, insbesondere bevorzugt 0,3 bis 20 Gew.-%, jeweils bezogen auf das Gesamtgewicht der kosmetischen Zusammensetzung.

Weitere bevorzugte Beispiele für Konditionierungsmittel sind Fettsäureester und Fettsäurealkohole.

Beispiele für Fettsäurealkohole sind Alkohole mit C8-C28 Kohlenstoffketten wie die Fettalkohole 1-Octadecanol mit dem INCI-Namen Stearyl Alcohol, 1-Hexadecanol mit dem INCI-Namen Cetyl Alcohol, oder Fettalkohole mit den INCI-Namen Cetearyl Alcohol, Myristyl Alcohol, Caprylic Alcohol, Lauryl Alcohol, Decyl Alcohol und Oleyl Alcohol.

Fettsäurealkohole erfüllen neben konditionierenden Eigenschaften auch eine strukturgebende, verdickende Wirkung bei kosmetischen Zusammensetzungen.

Weitere Beispiele für Fettsäureester sind Ester der Fettsäuren mit dem INCI-Namen Palmitic Acid, Oleic Acid, Linolic Acid, Linoleic Acid, Caprylic Acid, Myristic Acid und Stearic Acid, beispielsweise Fettsäureester mit dem INCI-Namen Isopropyl Palmitate, Ethylhexyl Palmitate, Isopropyl Myristate und Isopropyl Stearate.

Wenn Fettsäureester und Fettsäurealkohole eingesetzt werden, enthält die erfindungsgemäße kosmetische Zusammensetzung Fettsäureester und Fettsäurealkohole vorzugsweise in Mengen von 0,1 bis 15 **Gew.-%,** bevorzugt von 0,3 bis 12 **Gew.-%,** insbesondere bevorzugt 0,5 bis 10 **Gew.-%,** jeweils bezogen auf das Gesamtgewicht der kosmetischen Zusammensetzung.

Weitere bevorzugte Beispiele für Konditionierungsmittel sind natürliche oder synthetische Öle und Wachse.

Beispiele für bevorzugte Öle und Wachse sind Kohlenwasserstoffe mit linearen oder verzweigten, gesättigten oder ungesättigten C4-C60 Kohlenstoffketten, wie Öle und Wachse mit den INCI-Namen Isododecane, hydrated Polyisobutylene, hydrated Polydecene, Paraffin und Isoparaffin.

Weitere Beispiele für bevorzugte Öle und Wachse sind Carnaubawachs, Bienenwachs, Wollwachs, mikrokristallines Wachs, Jojobaöl, Reisöl, Calendulaöl, Sonnenblumenöl, Sojaöl, Kokosnussöl, Olivenöl und Mandelöl.

Wenn natürliche oder synthetische Öle und Wachse eingesetzt werden, enthält die erfindungsgemäße kosmetische Zusammensetzung Öle und Wachse vorzugsweise in Mengen von 0,1 bis 10 Gew.-%, bevorzugt von 0,2 bis 7 Gew.-%, insbesondere bevorzugt 0,3 bis 5 Gew.-%, jeweils bezogen auf das Gesamtgewicht der kosmetischen Zusammensetzung.

Weitere bevorzugte Beispiele für Konditionierungsmittel sind Panthenol, Lipide, wie Ceramide, Proteine und hydrolysierte Proteine, wie hydrolisiertes Kollagen, hydrolysierte Weizenproteine und hydrolysierte Seide.

Optional enthält die kosmetische Zusammensetzung weitere kosmetisch übliche Additive, wie z.B. Tenside, Verdicker, Gelierungsmittel, Filmbildner, feuchtigkeitsspendende Mittel, UV-Filter, Perlglanzpigmente, Vitamine, Antioxidantien, Coffein, Anti-Schuppenwirkstoffe oder Konservierungsmittel.

Beispiele für weitere in der Kosmetik übliche Additive und deren INCI-Namen sind beschrieben im "International Cosmetic Ingredient Dictionary & Handbook" des Personal Care Product Council.

Optional enthält die kosmetische Zusammensetzung weitere kosmetisch übliche Additive wie Tenside.

Beispiele für in der Kosmetik übliche Tenside sind auch in K. Schrader, A. Domsch, Cosmetology - Theory and Practice, Volume II, Seite II-8 bis II-22, Verlag für chemische Industrie, 2005, sowie in Punkt 1. bis 18. unter Beispiele für Emulgatoren, beschrieben.

Beispiele für vorzugsweise eingesetzte anionische Tenside entsprechen den in Punkt 1. bis 3. unter Beispiele für anionische Emulgatoren aufgeführten Materialien.

INCI-Namen von vorzugsweise eingesetzten anionischen Tensiden sind beispielsweise Sodium Lauryl Sulfate, Ammonium Laureth Sulfate, Sodium Laureth Sulfate, Disodium 2-Sulfolaurate, Disodium Lauryl Sulfosuccinate oder Disodium Laureth-Sulfosuccinate.

Wenn anionische Tenside eingesetzt werden, enthält die erfindungsgemäße kosmetische Zusammensetzung anionische Tenside vorzugsweise in Mengen von 1 bis 30 Gew.-%, bevorzugt von 5 bis 25 Gew.-%, insbesondere bevorzugt 7 bis 20 Gew.-%, jeweils bezogen auf das Gesamtgewicht der kosmetischen Zusammensetzung.

Beispiele für vorzugsweise eingesetzte nichtionische Tenside entsprechen den in Punkt 5. bis 13. unter Beispiele für nichtionische Emulgatoren aufgeführten Materialien.

INCI-Namen von vorzugsweise eingesetzten nichtionischen Tensiden sind beispielsweise Coco Glucoside, Lauryl glucoside, Decyl Glucoside, PEG-40 Hydrogenated Castor Oil, Polysorbate 80 oder PEG-7 Glyceryl Cocoate.

Wenn nichtionische Tenside eingesetzt werden, enthält die erfindungsgemäße kosmetische Zusammensetzung nichtionische Tenside vorzugsweise in Mengen von 1 bis 15 Gew.-%, bevorzugt von 2 bis 12 Gew.-%, insbesondere bevorzugt 3 bis 10 Gew.-%, jeweils bezogen auf das Gesamtgewicht der kosmetischen Zusammensetzung.

Beispiele für vorzugsweise eingesetzte amphotere Tenside entsprechen den in Punkt 17. bis 18. unter Beispiele für nichtionische Emulgatoren aufgeführten Materialien. Weitere bevorzugte Beispiele sind Verbindungen aus den Klassen der Alkylamidobetaine, Alykylamphoacetate und Alkylamphopropionate. INCI-Namen von vorzugsweise eingesetzten nichtionischen Tensiden sind beispielsweise Cocamidopropyl Betaine, Cetyl Betaine, Cocamide MEA, Cocamide DEA, Cocamide MIPA, Sodium Cocoamphoacetate oder Sodium Cocoamphopropionate.

Wenn amphotere Tenside eingesetzt werden, enthält die erfindungsgemäße kosmetische Zusammensetzung amphotere Tenside vorzugsweise in Mengen von 1 bis 15 Gew.-%, bevorzugt von 2 bis 12 Gew.-%, insbesondere bevorzugt 3 bis 10 Gew.-%, jeweils bezogen auf das Gesamtgewicht der kosmetischen Zusammensetzung.

Optional enthält die kosmetische Zusammensetzung weitere kosmetisch übliche Additive wie Verdicker.

Beispiele für vorzugsweise eingesetzte Verdicker sind modifizierte Polysaccharide wie Stärke, Cellulose, Gummi Arabicum und Guar Gums, z.B. Polmyere mit dem INCI-Namen Cellulose Gum, Guar Gum, Xanthan Gum oder Cassia Gum.

Weitere Beispiele für Verdicker sind hydrophob modifizierte nichtionische Cellulosederivate, z.B. das Cellulosederivat mit dem INCI-Namen Hydroxyethylcellulose.

Weitere Beispiele für Verdicker sind vernetzte Acrylsäure- und Methacrylsäurepolymere und Derivate der vernetzten Acrylsäure- und Methacrylsäurepolymere, z.B. Polymere mit dem INCI-Namen Carbomer.

Weitere Beispiele für Verdicker sind Agentien, die in Kombination mit Tensiden eine verdickende Wirkung erzielen. Beispiele sind Monoglyceride von Fettsäuren, Mono/Diglyceride von ethoxylierten Fettsäuren und ethoxylierte Fettalkohole. INCI-Namen von vorzugsweise eingesetzten Verdickern, die in Kombination mit Tensiden eine verdickende Wirkung erzielen, sind PEG-120 Methyl Glucose Dioleate, PEG-150 Distearate, Myristyl Glycol, PEG-200 Glyceryl Palmitate, Laureth-4 oder PEG-200 Glyceryl Palmitate.

Weitere Beispiele für Verdicker sind Salze, z.B. Salze mit dem INCI-Namen Sodium Chloride.

Wenn Verdicker eingesetzt werden, enthält die erfindungsgemäße kosmetische Zusammensetzung Verdicker vorzugsweise in Mengen von 0,1 bis 10 Gew.-%, jeweils bezogen auf das Gesamtgewicht der kosmetischen Zusammensetzung.

Optional enthält die kosmetische Zusammensetzung weitere kosmetisch übliche Additive wie Filmbildner.

Bevorzugte Beispiele für Filmbildner sind Polymere.

Beispiele für vorzugsweise eingesetzte filmbildende Polymere sind beschrieben im "International Cosmetic Ingredient Dictionary & Handbook" des Personal Care Product Councils.

Beispiele für bevorzugte filmbildende Polymere sind Acrylsäurepolymerderivate, Acrylsäurecopolymerderivate, Methacrylsäurederivate und Methacrylsäurecopolymerderivate.

Beispiele für bevorzugte anionische Polymere sind Copolymere aus Vinyl Acetate und einem oder mehrerer Acrylsäure-, Methacrylsäuremonomere und deren Ester, z.B. Polymere mit dem INCI-Namen Acrylates/VA Copolymer.

Weitere Beispiele für bevorzugte filmbildende Polymere sind Copolymere aus Vinylpyrrolidone und einem oder mehrerer Acrylsäure-, Methacrylsäuremonomere und deren Ester, z.B Polymere mit dem INCI-Namen Acrylates/VP Copolymer.

Weitere Beispiele für bevorzugte filmbildende Polymere sind Copolymere aus tert-Butyl Acrylamide und einem oder mehrerer Acrylsäure-, Methacrylsäuremonomere und deren Ester, z.B. Polymere mit dem INCI-Namen Acrylates/t-Butylacrylamide Copolymer.

Weitere Beispiele für bevorzugte filmbildende Polymere sind Copolymere aus Vinyl Acetate, Crotonsäure und Vinyl Neodecanoate Monomeren, z.B. Polymere mit dem INIC-Namen VA/Crotonates/Vinyl Neodecanoate Copolymer.

Weitere Beispiele für bevorzugte filmbildende Polymere sind Copolymere aus Vinyl Acetate, Crotonsäure und Vinyl Neodecanoate Monomeren und Vinylsiliconen, z.B. Polymere mit dem INCI-Namen Crotonic Acid/Vinyl C8-C12 Isoalkyl Esters/VA/Bis-Vinyldimethicone Copolymer.

Wenn filmbildende Polymere eingesetzt werden, enthält die erfindungsgemäße kosmetische Zusammensetzung filmbildende Polymere vorzugsweise in Mengen von 0,1 bis 15 Gew.- %, bevorzugt von 0,2 bis 10 Gew.-%, insbesondere bevorzugt 0,3 bis 7 Gew.-%, jeweils bezogen auf das Gesamtgewicht der kosmetischen Zusammensetzung.

Optional enthält die kosmetische Zusammensetzung weitere kosmetisch übliche Additive wie feuchtigkeitsspendende Mittel.

Beispiele für vorzugsweise eingesetzte feuchtigkeitsspendende Mittel sind Glycerin, Sorbitol, Xylitol, Polyethylenglycol, 1,2-Propandiol, 1,3-Propandiol oder Polypropylenglykol.

Wenn feuchtigkeitsspendende Mittel eingesetzt werden, enthält die erfindungsgemäße kosmetische Zusammensetzung feuchtigkeitsspendende Mittel vorzugsweise in Mengen von 0,1 bis 10 Gew.-%, bevorzugt von 0,2 bis 8 Gew.-%, insbesondere bevorzugt 0,3 bis 6 Gew.-%, jeweils bezogen auf das Gesamtgewicht der kosmetischen Zusammensetzung.

Optional enthält die kosmetische Zusammensetzung weitere kosmetisch übliche Additive wie perlglanzgebende Mittel.

Beispiele für vorzugsweise eingesetzte perlglanzgebende Mittel sind Perlglanzpigmente oder Glycol Distearate.

Wenn Perlglanz gebende Mittel eingesetzt werden, enthält die erfindungsgemäße kosmetische Zusammensetzung Perlglanz gebende Mittel vorzugsweise in Mengen von 0,1 bis 7 Gew.-%, bevorzugt von 0,2 bis 6 Gew.-%, insbesondere bevorzugt 0,3 bis 5 Gew.-%, jeweils bezogen auf das Gesamtgewicht der kosmetischen Zusammensetzung.

Die kosmetischen Zusammensetzungen werden vorzugsweise hergestellt durch Mischen mindestens einer erfindungsgemäßen wässrigen Dispersion von vorvernetzten Organopolysiloxanen mit gegebenenfalls mindestens einem Konditionierungsmittel und ggf. weiteren kosmetisch üblichen Additiven in einem kosmetisch akzeptablen Medium, bevorzugt Wasser.

Die einzelnen Inhaltsstoffe können in einem heiß/heiß-, heiß/kalt- oder kalt/kalt-Verfahren miteinander vermischt werden.

Die Zugabe der erfindungsgemäßen Dispersionen von vorvernetzten Organopolysiloxanen beim Herstellen der erfindungsgemäßen kosmetischen Zusammensetzung erfolgt vorzugsweise bei Temperaturen von höchstens 50°C, bevorzugt bei Temperaturen von höchstens 40°C, besonders bevorzugt bei Temperaturen von höchstens 35°C. Sie erfolgt vorzugsweise bei Temperaturen von mindestens 5°C, bevorzugt bei Temperaturen von mindestens 10°C.

Die erfindungsgemäße kosmetische Zusammensetzung kann vorliegen in Form einer Emulsion, einer Suspension, einer Lösung, einer Creme, einer Lotion, eines Schaums, eines Stifts, eines Seifenstücks, einer Paste, eines Gels.

Die erfindungsgemäße kosmetische Zusammensetzung in Form einer Emulsion kann vorliegen in Form einer W/O-Emulsion (Wasser-in-Öl-Emulsion), einer O/W-Emulsion (Öl-in-Wasser-Emulsion) oder als multiple Emulsion.

Ist das Ziel, eine kosmetische Zusammensetzung enthaltend eine erfindungsgemäße wässrige Dispersion von vorvernetzten Organopolysiloxanen in Form einer Emulsion in transluzenter oder transparenter Erscheinungsform herzustellen, werden bevorzugt erfindungsgemäße wässrige Dispersionen von vorvernetzten Organopolysiloxanen mit Teilchengrößen < 700 nm, bevorzugt mit Teilchengrößen < 400 nm, insbesondere bevorzugt mit Teilchengrößen < 300 nm, eingesetzt.

Ein weiterer Gegenstand der Erfindung ist die Verwendung der erfindungsgemäßen kosmetischen Zusammensetzung zur Behandlung keratinischer Fasern, wie Haaren. Vorzugsweise werden die kosmetischen Zusammensetzungen zur Reinigung und Pflege von keratinischen Fasern, wie Haaren, oder zur Formgebung von keratinischen Fasern, wie Haaren, verwendet.

Beispiele für Mittel zur Reinigung und Pflege von Haaren sind Haar-Shampoos, -Spülungen (Rinse-Off Conditioner), -Kuren, -Masken, -Seren, -Schäume, -Stylingsprays, -Cremes, -Gele, -Öle, -Spitzenfluids und -Färbemittel.

Ein weiterer Gegenstand der Erfindung ist die Verwendung der erfindungsgemäßen kosmetischen Zusammensetzung zum Konditionieren von keratinischen Fasern, wie Haaren, um insbesondere die Kämmbarkeit zu erleichtern.

Ein weiterer Gegenstand der Erfindung ist ein Verfahren zur Behandlung von keratinischen Fasern, vorzugsweise Haaren, indem die erfindungsgemäßen kosmetischen Zusammensetzungen auf die keratinischen Fasern, vorzugsweise Haare, aufgetragen werden und dann gegebenenfalls mit Wasser gespült werden.

In den nachstehend beschriebenen Beispielen beziehen sich alle Angaben von Teilen und Prozentsätzen, falls nicht anders angegeben, auf das Gewicht. Des Weiteren beziehen sich alle Viskositätsangaben auf eine Temperatur von 25°C. Sofern nichts anders angegeben, werden die nachstehenden Beispiele bei einem Druck der umgebenden Atmosphäre, also etwa 1020 hPa, und bei Raumtemperatur, also bei etwa 20°C, bzw. bei einer Temperatur, die sich beim Zusammengeben der Reaktanten bei Raumtemperatur ohne zusätzliche Heizung oder Kühlung einstellt, durchgeführt.


**Synthese der Oxalamido-ethylester-terminierten Organopolysiloxane S1-S2**


**Synthese des Oxalamido-ethylester-terminierten Organopolysiloxans S1**


Eine Apparatur bestehend aus einem 4-l Dreihalskolben mit Magnetrührstäbchen, Magnetrührer, Rückflusskühler, Innenthermometer und Tropftrichter wurde mit Stickstoffgas inertisiert. Anschließend wurden unter kontinuierlichem Stickstoffstrom 2679 g (18,45 mol) Diethyloxalat (M = 146,14 g/mol; CAS-Nr. 95-92-1; erhältlich bei Sigma-Aldrich, München, Deutschland) vorgelegt und unter Rühren kontinuierlich 600 g (4,07 mol) (3-Aminopropyl)dimethylmethoxysilan (M = 147,29 g/mol; CAS-Nr. 31024-26-7; erhältlich bei Gelest, Morrisville, USA) so zugetropft, dass die Temperatur der Reaktionsmischung stets unter 50°C blieb. Dabei entstand eine schwach gelbliche, klare Flüssigkeit, die nach Ende der Dosierung eine Stunde bei Raumtemperatur weitergerührt wurde. Anschließend wurde das überschüssige Diethyloxalat unter Vakuum (2 mbar) abdestilliert. Man erhielt 1995 g Diethyloxalat (13,65 mol) als farblose, klare Flüssigkeit. Der flüssige Destillationsrückstand wurde bei einer Innentemperatur von 160°C fraktioniert destilliert. Innerhalb des Siedebereichs von 136 bis 141°C / 2 mbar Vakuum destillierten 949 g (3,84 Mol) des gewünschten Hauptprodukts Ethyl 2-((3-(methoxydimethylsilyl)propyl)amino)-2-oxoacetat (EtO-CO-CO-HN-CH$_2$CH$_2$CH$_2$-Si-Me$_2$(OMe)) **P1** (M = 247,37 g/mol) als farblose, klare Flüssigkeit. Die Ausbeute betrug 94% bezogen auf das eingesetzte (3-Aminopropyl)dimethylmethoxysilan. 164,95 g (0,66 mol) **P1** und 740 g (10,00 mol Si) $\alpha,\omega$-OH-terminiertes, lineares Dimethylsiloxan (CAS-Nr. 70131-67-8) mit einer Viskosität von 50 mPas wurden in einem 2-l Rundkolben vorgelegt. Der Rundkolben mit dem Reaktionsgemisch wurde am Rotationsverdampfer in einem 130°C heißen Ölheizbad bei einem Vakuum von 6 mbar 30 Minuten lang unter Rotation erhitzt. Anschließend wurde das Vakuum gebrochen, 0,02 g einer 40%igen Lösung von PNCl$_2$ in Ethylacetat zugegeben und eine Stunde am Rotationsverdampfer unter Vakuum erhitzt (130 °C Ölbad, 6 mbar Vakuum). Danach wurde das Vakuum erneut gebrochen, 0,02 g einer 40%igen Lösung von PNCl$_2$ in Ethylacetat (10 ppm PNCl$_2$) zugegeben und eine Stunde am Rotationsverdampfer weiter unter Vakuum erhitzt (130 °C Ölbad, 6 mbar Vakuum). Anschließend wurde wiederum das Vakuum gebrochen, 0,02 g einer 40%igen Lösung von PNCl$_2$ in Ethylacetat (10 ppm PNCl$_2$) zugegeben und 4 Stunden am Rotationsverdampfer unter Vakuum erhitzt (130 °C Ölbad, 6 mbar Vakuum). Nach Abkühlen auf Raumtemperatur wurden dem Kolbeninhalt 8 g Magnesiumoxid (MgO schwer; CAS-Nr.

1309-48-4; erhältlich bei Sigma-Aldrich, München, Deutschland) zugegeben und 1 h bei Raumtemperatur gerührt. Die Suspension wurde anschließend unter 6 mbar Druck über einen Seitz® T-120 Tiefenfilter (erhältlich bei Pall Corporation, New York, USA) filtriert. Man erhielt 793 g eines farblosen, klaren Öls und einer Viskosität von ~95 mPas.

**Synthese des Oxalamido-ethylester-terminierten Organopolysiloxans S2**

In einer Rührkolben-Apparatur bestehend aus 1-l Dreihalskolben mit Heizpilz, mechanischem Rührer, Innenthermometer und Wasserabscheider mit Rückflusskühler wurden 400 g (5,41 mol Si) $\alpha,\omega$-OH-terminiertes, lineares Dimethylsiloxan (CAS-Nr. 70131-67-8) mit einer Viskosität von 50 mPas sowie 19,8 g (0,08 mol) **P1** vorgelegt und unter Rühren auf 80°C erhitzt. Nach Zugabe von 50 mg PNCl$_2$ (100%ig) wurde Vakuum (10 mbar) angelegt und der Kolbeninhalt auf 100 °C aufgeheizt. Das dabei entstehende Wasser wurde über den Wasserabscheider entfernt. Nach 5 Minuten wurde ein Vakuum von 40 mbar eingestellt und 30 Minuten lang unter diesen Bedingungen gerührt. Danach wurde das Vakuum gebrochen, weitere 25 mg PNCl$_2$ (100%ig) zugegeben und bei 100°C und 40 mbar unter Rühren erhitzt. Anschließend wurde das Vakuum erneut gebrochen, 25 mg PNCl$_2$ (100%ig) zugegeben und bei 100°C und 40 mbar unter Rühren weiter erhitzt. Im Anschluss wurde das Vakuum gebrochen, der Ansatz auf 70-80°C Innentemperatur abgekühlt und der Kolbeninhalt unter Zugabe von 2,5 g wasserfreier Soda (Natriumcarbonat leicht; CAS-Nr. 497-19-8; erhältlich bei Sigma-Aldrich, München, Deutschland) neutralisiert. Es wurden 30 Minuten weitergerührt und anschließend filtriert. Man erhielt ein farbloses, klares Öl mit einer Viskosität von ~525 mPas.

**Herstellung der Aminosiliconöl-Emulsionen E1 - E3:**

**Herstellung der Aminosiliconöl-Emulsion E1:**

Mit einem Ultra-Turrax T 50 Emulgiergerät (Fa. Janke & Kunkel / IKA) werden bei 4000 U/min 4,9 g einer 80%-igen wässrigen Lösung von Isotridecyldecaethoxylat, käuflich erwerblich unter dem Handelsnamen Lutensol TO 10 (Fa. BASF) und 1,6 g vollentsalztes Wasser vorgemischt. 34,9 g eines hydroxy/methoxyterminierten Copolymers aus 3-(2-Aminoethylamino)propyl-methylsiloxy- und Dimethylsiloxy-einheiten mit einer Aminzahl von 0,14 meq/g und einer Viskosität von 4000 mm$^2$/s (bei 25°C) werden in drei Portionen bei einer Scherung von 4000 U/min zugegeben, so dass eine relativ feste steife Phase als Voremulsion resultiert. Es wird mit 57,5 g vollentsalztem Wasser portionsweise unter geringer Scherung zur gewünschten Emulsion verdünnt und mit 0,20 g 80%-iger Essigsäure und mit 0,9 g 2-Phenoxyethanol versetzt.
Man erhält eine glatte, niederviskose, weiße Siliconöl-Emulsion **E1** mit einem Festgehalt von 39,8% und einem pH-Wert von 5,0. Eine Messung der Teilchengrößenverteilung ergibt einen D50-Wert von 160 nm.

**Herstellung der Aminosiliconöl-Emulsion E2:**

Im Arbeitsgefäß eines Laborplanetenmischers (Typ Labmax, Fa. Molteni) werden bei 500 U/min 30,0 g einer 80%-igen wässrigen Lösung von Isotridecyldecaethoxylat, käuflich erwerblich unter dem Handelsnamen Lutensol TO 10 (Fa. BASF), 81,7 g eines trimethylsilyl-terminierten Copolymers aus 3-(2-Amino-ethylamino)propyl-methylsiloxy- und Dimethylsiloxy-einheiten mit einer Aminzahl von 0,6 meq/g und einer Viskosität von 2500 mm$^2$/s (bei 25°C) und 86,3 g eines trimethylsilyl-terminierten Polydimethylsiloxans mit einer Viskosität von 60000 mm$^2$/s (bei 25°C) vorgemischt. Nach Zugabe von 39,6 g vollentsalztem Wasser und 1,4 g 80%-iger Essigsäure (bei 500 U/min) wird in drei Zyklen bei einer Scherung von 2500 U/min homogenisiert, so dass eine weiche bis mittelfeste steife Phase als Voremulsion resultiert. Es wird mit 157,4 g vollentsalztem Wasser portionsweise unter geringerer Scherung zur gewünschten Emulsion verdünnt und diese mit 3,6 g 2-Phenoxyethanol versetzt.
Man erhält eine glatte, fließende, weiße Siliconöl-Emulsion **E2** mit einem Festgehalt von 48,9% und einem pH-Wert von 5,5.
Eine Messung der Teilchengrößenverteilung ergibt einen D50-Wert von 180 nm.

**Herstellung der Aminosiliconöl-Emulsion E3:**

Im Arbeitsgefäß eines Laborplanetenmischers (Typ Labmax, Fa. Molteni) werden bei 500 U/min 32,0 g einer 80%-igen wässrigen Lösung von Isotridecyldecaethoxylat, käuflich erwerblich unter dem Handelsnamen Lutensol TO 10 (Fa. BASF), 44,0 g eines hydroxy/methoxy-terminierten Copolymers aus 3-(2-Aminoethylamino)propyl-methylsiloxy- und Dimethylsiloxy-einheiten mit einer Aminzahl von 0,14 meq/g und einer Viskosität von 4000 mm$^2$/s (bei 25°C) und 132,0 g eines trimethylsilyl-terminierten Polydimethylsiloxans mit einer Viskosität von 60000 mm$^2$/s (bei 25°C) vorgemischt. Nach Zugabe von 20,0 g vollentsalztem Wasser und 0,3 g 80%-iger Essigsäure (bei 500 U/min) wird in drei Zyklen bei einer Scherung von 2500 U/min homogenisiert, so dass eine feste steife Phase als Voremulsion resultiert. Es wird mit 168,1 g vollentsalztem Wasser portionsweise unter geringerer Scherung zur gewünschten Emulsion verdünnt und diese mit 3,6 g 2-Phenoxyethanol versetzt.

Man erhält eine glatte, dünnflüssige, weiße Siliconöl-Emulsion **E3** mit einem Festgehalt von 51,3% und einem pH-Wert von 5,0. Eine Messung der Teilchengrößenverteilung ergibt einen D50-Wert von 257 nm.

**Beispiele 1-4:**

Die nachfolgenden Beispiele **1-4** repräsentieren Herstellungsverfahren zur Synthese von erfindungsgemäßen wässrigen Dispersionen von vorvernetzten Organopolysiloxanen, die zur Herstellung von erfindungsgemäßen kosmetischen Zusammensetzungen eingesetzt werden.

**Beispiel 1**

```
Emulsion B1-a = B1-b
```

Mit einem Ultra-Turrax T 50 Emulgiergerät (Fa. Janke & Kunkel / IKA) werden bei 5000 U/min 92,26 g der Aminosiliconöl-Emulsion **E1** mit 7,74 g Oxalamido-ethylester-terminiertem Organopolysiloxan **S1** innerhalb einer Minute homogenisiert.

Man erhält eine glatte, niederviskose, weiße Siliconöl-Emulsion **B1-a (= B1-b)** mit einem Festgehalt von 44,5% und einem pH-Wert von 5,0. Eine Messung der Teilchengrößenverteilung ergibt einen D50-Wert von 141 nm.

Durch Eindampfen der Emulsion wird nach einer Trocknungszeit von 24 Stunden bei 25°C ein ausgeprägter, weißlicher, etwas weicher, elastischer, auf der Oberfläche etwas klebriger Film erhalten.

**Emulsion B1-c**

Mit einem Ultra-Turrax T 50 Emulgiergerät (Fa. Janke & Kunkel / IKA) werden bei 5000 U/min 96,16 g der Aminosiliconöl-Emulsion **E1** mit 3,84 g Oxalamido-ethylester-terminiertem Organopolysiloxan **S1** innerhalb einer Minute homogenisiert.

Man erhält eine glatte, niederviskose, weiße Siliconöl-Emulsion **B1-c** mit einem Festgehalt von 42,1% und einem pH-Wert von 5,5. Eine Messung der Teilchengrößenverteilung ergibt einen D50-Wert von 123 nm.

Durch Eindampfen der Emulsion wird nach einer Trocknungszeit von 24 Stunden bei 25°C ein ausgeprägter, weißlicher, etwas weicher, elastischer Film erhalten.

**Beispiel 2**

**Emulsion B2**

Mit einem Ultra-Turrax T 50 Emulgiergerät (Fa. Janke & Kunkel / IKA) werden bei 5000 U/min 96,08 g der Aminosiliconöl-Emulsion **E2** mit 3,92 g Oxalamido-ethylester-terminiertem Organopolysiloxan **S1** innerhalb einer Minute homogenisiert.

Man erhält eine glatte, fließende, weiße Siliconöl Emulsion **B2** mit einem Festgehalt von 50,9% und einem pH-Wert von 6,5. Eine Messung der Teilchengrößenverteilung ergibt einen D50-Wert von 230 nm.

Durch Eindampfen der Emulsion wird nach einer Trocknungszeit von 24 Stunden bei 25°C ein ausgeprägter, weißlicher, etwas weicher, elastischer Film erhalten.

**Beispiel 3**

**Emulsion B3**

Mit einem Ultra-Turrax T 50 Emulgiergerät (Fa. Janke & Kunkel / IKA) werden bei 5000 U/min 98,22 g der Aminosiliconöl-Emulsion **E3** mit 1,78 g Oxalamido-ethylester-terminiertem Organopolysiloxan **S1** innerhalb einer Minute homogenisiert.

Man erhält eine glatte, dünnflüssige, weiße Siliconöl-Emulsion **B3** mit einem Festgehalt von 52,2% und einem pH-Wert von 4,5. Eine Messung der Teilchengrößenverteilung ergibt einen D50-Wert von 210 nm.

Durch Eindampfen der Emulsion wird nach einer Trocknungszeit von 24 Stunden bei 25°C ein ausgeprägter, weißlicher, etwas weicher, elastischer, auf der Oberfläche etwas klebriger Film erhalten.

**Beispiel 4**

**Emulsion B4**

Mit einem Ultra-Turrax T 50 Emulgiergerät (Fa. Janke & Kunkel / IKA) werden bei 5000 U/min 85,69 g der Aminosiliconöl-Emulsion **E1** mit 14,31 g Oxalamido-ethylester-terminiertem Organopolysiloxan **S2** innerhalb einer Minute homogenisiert.

Man erhält eine glatte, niederviskose, weiße Siliconöl-Emulsion **B4** mit einem Festgehalt von 48,4% und einem pH-Wert von 5,5. Eine Messung der Teilchengrößenverteilung ergibt einen D50-Wert von 192 nm.

Durch Eindampfen der Emulsion wird nach einer Trocknungszeit von 24 Stunden bei 25°C ein ausgeprägter, weißlicher, etwas weicher, elastischer Film erhalten.

**Vergleichsversuch V1:**

Der nachfolgende Vergleichsversuch **V1** repräsentiert ein Herstellverfahren zur Synthese von wässrigen, nicht-erfindungsgemäßen Emulsionen (Vernetzung mit nicht-erfindungsgemäßem Diethyloxalat gemäß US5039738 A):

Mit einem Ultra-Turrax T 50 Emulgiergerät (Fa. Janke & Kunkel / IKA) werden bei 5000 U/min 99,6 g der Aminosiliconöl-Emulsion **E1** mit 0,4 g Diethyloxalat innerhalb einer Minute homogenisiert.

Man erhält eine glatte, niederviskose, weiße Siliconöl-Emulsion **V1** mit einem Festgehalt von 40,0% und einem pH-Wert von 4,5. Eine Messung der Teilchengrößenverteilung ergibt einen D50-Wert von 71 nm.

Durch Eindampfen der Emulsion wird nach einer Trocknungszeit von 24 Stunden bei 25 °C eine weiß-opake, auf Glas und Aluminium schwach haftende, pastöse Schicht erhalten.

**Vergleichsversuch V2:**

Der nachfolgende Vergleichsversuch **V2** repräsentiert ein Herstellverfahren zur Synthese von wässrigen, nicht-erfindungsgemäßen Emulsionen (Vernetzung mit nicht-erfindungsgemäßem Diethyl-L-Tartrat: gemäß WO20239229 A1):

Mit einem Ultra-Turrax T 50 Emulgiergerät (Fa. Janke & Kunkel / IKA) werden bei 5000 U/min 99,4 g der Aminosiliconöl-Emulsion **E1** mit 0,6 g Diethyl-L-Tartrat innerhalb einer Minute homogenisiert.

Man erhält eine glatte, niederviskose, weiße Siliconöl-Emulsion **V2** mit einem Festgehalt von 40,2% und einem pH-Wert von 5,5. Eine Messung der Teilchengrößenverteilung ergibt einen D50-Wert von 135 nm.

Durch Eindampfen der Emulsion wird nach einer Trocknungszeit von 24 Stunden bei 25°C ein weicher, milchig-weißer, elastischer Film mit leicht klebriger Oberfläche erhalten.

**Rheologie der Elastomerfilme nach Entfernen von Wasser:**

Auf eine Glas-Rundplatte mit 65 mm Durchmesser wird ein Teflonring mit 40 mm Innendurchmesser gelegt, um so eine Gussform zu bilden. In diesen Ring werden 2,5 g der zu vermessenden Emulsion auf die Glasplatte blasenfrei eingewogen. Anschließend lässt man die gegossene Emulsion bei 25°C und 101,425 kPa trocknen. Es ist darauf zu achten, dass sich die Gussform mit der gegossenen Emulsion zum Trocknen auf einer nivellierten ebenen Fläche befindet, damit sich ein gleichmäßig dicker Film ausbilden kann. Man erhält hier nach der Trocknung einen ca. 0,5 mm dicken Film.

Nach 1 Woche Standzeit bei 25°C werden die erhaltenen Filme rheologisch untersucht. Die Messungen erfolgten mit einem Rheometer "MCR 302" der Firma Anton Paar, wobei ein Platte-Platte-Messsystem PP12.5 bei einer Spalthöhe von 0,5 mm verwendet wurde. Die Kalibrierung des Gerätes erfolgte mit Normalöl 10000 der Physikalisch-Technischen Bundesanstalt. Die Messtemperatur beträgt 25,00°C +/- 0,05°C.

Die in Tabelle 1 aufgeführten Werte des Speichermoduls G', Verlustmoduls G'', und des Verlustfaktors tan $\delta$ können errechnet werden, indem durch Vorgabe einer sinusförmigen Deformation die Schubspannung $\tau$ und der Phasenverschiebungswinkel $\delta$ gemessen werden. Die in Tabelle 1 aufgeführten Messwerte wurden bei einer Frequenz von 1 Hz und bei einer Deformation von 0.1% gemessen. Bei einer Deformation liegen die gemessenen Proben im linear visko-elastischen Messbereich. Dabei gilt: tan $\delta$ = G''/G'. Wenn tan $\delta$ < 1 überwiegt der elastische Charakter der Probe, wenn tan $\delta$ > 1 überwiegt der viskose Charakter der Probe.

Die Ergebnisse der Rheologiemessungen für die Elastomerfilme der erfindungsgemäßen Beispiele **B1** bis **B4,** der Vergleichsversuche **V1** und **V2** sowie der unvernetzten Aminosiliconöl-Emulsionen **E1** bis **E3** sind in Tabelle 1 zusammengefasst.

**Tabelle 1:** Rheologische Daten der Elastomerfilme

| Beisp./Vergl. | Speichermodul G' [Pa] | Verlustmodul G" [Pa] | tan δ |
|---|---|---|---|
| **B1-a = B1-b** | 8409 | 4094 | 0,49 |
| **B1-c** | 13853 | 3872 | 0,28 |
| **B2** | 3465 | 997 | 0,29 |
| **B3** | 1881 | 1127 | 0,60 |
| **B4** | 3386 | 1676 | 0,50 |
| **V1** | 3182 | 3904 | 1,23 |
| **V2** | 17196 | 5809 | 0,34 |
| **E1** | 28 | 205 | 7,28 |
| **E2** | 14 | 138 | 9,99 |
| **E3** | 140 | 502 | 3,60 |

Tabelle 1 zeigt, dass die erfindungsgemäßen Emulsionen **B1** bis B4 mit Oxalamido-ethylester-terminierten Organopolysiloxanen **S1** bzw. **S2** als Vernetzerkomponente nach Entfernen von Wasser elastische Filme bilden, da tan δ < 1.

Die nicht-erfindungsgemäße Emulsion **V1** zeigt nach Entfernen von Wasser keine elastische Filmbildung, da tan δ größer 1 ist.

Die nicht-erfindungsgemäße Emulsion **V2** mit Diethyl-L-Tartrat als Vernetzerkomponente, zeigt nach Entfernen von Wasser gemäß WO20239229 A1 die Ausbildung eines elastischen Films, da tan δ < 1.

Die eingedampften, reinen Aminosiliconöl-Emulsionen **E1** bis **E3** zeigen nach dem Entfernen des Wassers keine Filmbildung, da tan δ in allen Fällen deutlich größer 1 ist. Dies zeigt, dass die Zugabe eines Vernetzers erforderlich ist, um eine Filmbildung zu erreichen.

***Testmethoden zur Beurteilung der Wirkung von kosmetischen Zusammensetzungen:***

*Naturhaar*

Die Beurteilung des Applikationsverhaltens der kosmetischen Zusammensetzung und deren Wirkung bezüglich Kämmkraft und Weichheit erfolgte auf kaukasischem Haar, erhältlich bei der Firma Kerling International Haarfabrik GmbH. Ungeschädigte Naturhaartressen werden vor ihrem Einsatz gereinigt und in einem weiteren Prozessschritt eventuell durch Bleichen geschädigt.

*Grundreinigung*

Zur Reinigung werden die ungeschädigten Haartressen eine Stunde in eine Lösung aus Methylisobutylketon eingelegt und geschüttelt. Nach Entfernen des Lösungsmittels werden die Haartressen jeweils zweimal mit 3 ml einer Ammonium Lauryl Sulfat Lösung (25%ig), STEPANOL(R) ALS 25, Fa. STEPAN Company und anschließend 30°C warmen, vollentsalztem Wasser gewaschen. Dabei werden die Tressen mit einem grobzinkigen Kamm entwirrt. Anschließend werden die Haartressen eine Stunde in einem großen Becherglas in vollentsalztes Wasser gelegt, entnommen, und nochmals unter fließendem, vollentsalztem Wasser gespült. Nach der Grundreinigung werden die Tressen vor ihrer Weiterverwendung mindestens 12 Stunden bei 23°C und 50% Luftfeuchte konditioniert und vor ihrer Verwendung gekämmt.

*Bleichen von Haaren - Erzeugung geschädigter Haare*

Geschädigte Haare werden durch Bleichen gereinigter Naturhaartressen (Fa. Kerling International Haarfabrik GmbH, Euro-Natur-Haar, Farbe 6/0, 20 cm, Tressengewicht 2 g) erzeugt. Hierzu werden jeweils fünf Haartressen für 30 Minuten in eine Lösung aus 30%igem Wasserstoffperoxid und 25%igem Ammoniak (Verhältnis 33,5:1) eingelegt. Anschließend werden die Haare sorgfältig mit vollentsalztem Wasser ausgespült und zweimal mit 3 ml einer Ammonium Lauryl Sulfat Lösung (25%ig), STEPANOL(R) ALS 25, Fa. STEPAN Company und 30°C warmen, vollentsalztem Wasser gewaschen. Danach werden die Haartressen eine Stunde in einem großen Becherglas in vollentsalztes Wasser gelegt, entnommen, und nochmals unter fließendem, vollentsalztem Wasser gespült. Vor einer weiteren Behandlung werden die gebleichten Tressen mindestens 12 Stunden bei 23°C und 50% Luftfeuchte konditioniert und vor ihrer Verwendung gekämmt.

*Kämmkraftmessung:*

Zur Bestimmung der Kämmkraft von nassem und trockenem Haar wurden gebleichte oder ungebleichte Haartressen (Fa. Kerling International Haarfabrik GmbH, Euro-Natur-Haar, Farbe 6/0, 20 cm, Tressengewicht 2 g) verwendet. Die Messung der Kämmkraft mit Doppelkammmethode nach Y. K. Kamath und Hans-Dietrich Weigmann, J. Soc. Cosmet. Chem., 37, 111-124, 1986 erfolgte mit einer Zug-Dehnungsmaschine Instron 3343. Zunächst wird die Nass- und Trockenkämmkraft entlang der Messstrecke an unbehandelten Haartressen bestimmt. Anschließend werden die Haartressen mit einer erfindungsgemäßen kosmetischen Zusammensetzung behandelt und die Kraftaufnahme beim Kämmvorgang bestimmt. Als Messwert wird die Reduktion der Kämmkraft entlang der Messstrecke (Arbeit) angegeben, die sich zwischen der behandelten und unbehandelten Haartresse ergibt. Es wird der Mittelwert aus fünf Haartressen gebildet. Die Angabe der Kämmkraftreduktion erfolgt in Prozent.

*Geschmeidigkeit/Weichheit (gemäß Zug-Prüfung):*

Zur Bestimmung der Weichheit des Haares wurden gebleichte oder ungebleichte Haartressen (Fa. Kerling International Haarfabrik GmbH, Euro-Natur-Haar, Farbe 6/0, 20 cm, Tressengewicht 2 g) verwendet. Die Bestimmung der Haarweichheit im trockenen Zustand erfolgte durch Einsatz einer Zug-Prüfmaschine Instron 3343, indem die benötigte Zugkraft mit den Parametern Biegesteifigkeit und Oberflächenrauigkeit des Haarbündels in Korrelation gesetzt wird. Diese beiden Parameter wiederum korrelieren mit der Haarweichheit. Zu diesem Zweck wurde eine unbehandelte Haartresse in eine Messanordnung eingespannt, die aus fünf versetzt gegenüberliegenden Stäben besteht. Die Form der Haartresse in dieser Ausgangsposition ist eine Art Doppel-S. Die Haartresse wird nach dieser Vorbereitung in einer Richtung aus der Messanordnung gezogen und die notwendige Kraft entlang der Messstrecke als Arbeit ausgewertet. Anschließend werden die Haartressen mit einer erfindungsgemäßen kosmetischen Zusammensetzung behandelt und die Kraftaufnahme beim Ziehen der Haartresse durch die Messanordnung entlang der Messstrecke bestimmt. Als Messwert wird die Reduktion der Zugkraft entlang der Messstrecke (Arbeit) angegeben, die sich zwischen der behandelten und unbehandelten Haartresse ergibt. Eine hohe Reduktion der Zugkraft (Arbeit) entspricht einem guten Weichgriff bzw. einer hohen Geschmeidigkeit. Es wird der Mittelwert aus fünf Haartressen gebildet.

*Weichheit (gemäß Panel-Test):*

Zur Beurteilung der Weichheit von Haartressen werden dessen haptische Eigenschaften durch Experten (trainierte Panelisten) beurteilt. Es erfolgt jeweils ein paarweiser Vergleich von Haartressen, z.B. der Vergleich von Shampoo-behandelten Haaren zu unbehandelten Haaren. Die Zahl der beurteilten Tressenpaare beträgt mindestens drei, die Zahl der Panelisten mindestens fünf. Die Evaluierung basierte auf Haartressen der Fa. Kerling International Haarfabrik GmbH (Euro-Natur-Haar, Farbe 6/0, 20 cm, Tressengewicht 2 g).

*Aufwaschprozedur Shampoo:*

Auf eine gereinigte, befeuchtete Haartresse wird 0,2 g Shampoo pro g Haar appliziert. Das Shampoo wird 30 Sekunden lang in Richtung der Haarspitzen einmassiert. Anschließend wird die Haartresse 30 s unter fließendem, vollentsalztem Wasser ausgespült und mit einem grobzinkigen Kamm entwirrt. Die Prozedur wird zweimal wiederholt. Beim letzten Mal wird der Spülprozess auf 60 s verlängert. Anschließend wird die Haartresse mindestens 12 h bei einer Luftfeuchte von 50% und einer Temperatur von 23°C getrocknet.

*Aufwaschprozedur Conditioner:*

Auf eine gereinigte, befeuchtete Haartresse wird 0,3 g Rinse-Off Conditioner / g Haar appliziert. Der Rinse-Off Conditioner wird 120 Sekunden lang in Richtung der Haarspitzen einmassiert. Anschließend wird die Haartresse 60 s unter fließendem, vollentsalztem Wasser ausgespült und mit einem grobzinkigen Kamm entwirrt. Die Prozedur wird wiederholt. Anschließend wird die Haartresse mindestens 12 h bei einer Luftfeuchte von 50% und einer Temperatur von 23°C getrocknet.

*Bestimmung der abgeschiedenen Menge Si auf der Haaroberfläche in ppm (Silicondeponierung):*

Zur Bestimmung der abgeschiedenen Menge an Silicon auf der Haaroberfläche wird ein energiedispersives Röntgenfluoreszenz-Spektrometer (AMETEK, XEPOS) genutzt. Die Haarbündel werden in einem speziell angefertigten Probenhalter platziert, der eine kreisrunde Messfläche von 12 mm Durchmesser aufweist. Die Haaroberfläche im Bereich der Messfläche ist glatt, die Haare sind parallel ausgerichtet. Die Probe wird unter Heliumatmosphäre durch Einsatz einer Palladiumröhre angeregt (17,05 kV, 2,0 mA). Die Anregungsdauer beträgt 300 s. Kontrollproben (Naturhaartressen) werden regelmäßig gemessen. Bei Abweichungen erfolgt eine Driftkorrektur mit Glastabletten. Als Kalibrierstandards wurden Haartressen verwendet, die mit Polydimethylsiloxan im Bereich 50 bis 2000 ppm beladen waren (Kontrolle durch Atomabsorptionsspektroskopie).

Zur Bestimmung der Effektivität der Siliconabscheidung wird zunächst die Menge Si in ppm eines gereinigten Haarbündels bestimmt = Blindwert. Anschließend wird das gleiche Haarbündel z.B. durch Waschen mit einem Shampoo behandelt. Die Menge an Si in ppm wird erneut bestimmt = Probenwert. Die abgeschiedene Menge an Si in ppm ergibt sich durch Durchführung der Subtraktion Probenwert - Blindwert. Jede Haartresse wird mittig auf der Vorderseite und Rückseite vermessen. Als Ergebnis wird der Mittelwert aus drei Haartressen angegeben.

*Simulation von Shampoowäschen durch Rühren von Haartressen in einer Tensidlösung:*

Viele Nutzer wünschen nach Anwendung eines Haarkonditionierenden Produktes eine Persistenz kosmetischer Effekte wie verbesserte Haarweichheit, Nasskämmkraftreduktion, Erhalt der Haarfarbe trotz mehrerer, darauffolgender Wäschen mit Shampoo. Zur Beurteilung der Persistenz kosmetischer Effekte nach Behandlung von Haaren mit einer erfindungsgemäßen kosmetischen Zusammensetzung wurde eine Methode entwickelt, die eine Simulation von aufeinanderfolgenden Shampoowäschen darstellt. Dazu wird eine behandelte Haartresse in einem 100 ml Schraubdeckelglas mit 50 ml einer auf 40°C temperierten, fünfprozentigen Lösung von Ammonium Lauryl Sulfat, erhalten durch Verdünnen von STEPANOL® ALS 25, Fa. STEPAN Company, versetzt und eine vorgegebene Zeit in einem auf 40°C temperierten Inkubationsschüttler (Fa Heidolph Unimax 1010 + Incubator 1000) mit einer Geschwindigkeit von 250 rpm geschüttelt. Nach dem Schütteln werden die Tressen eine Minute mit 30°C warmen, vollentsalztem Wasser gespült und getrocknet.

*Curl Retention Test bei hoher Luftfeuchtigkeit:*

Der Curl Retention Test bei hoher Luftfeuchtigkeit erlaubt die Beurteilung von Pflege- und Stylingprodukten im Hinblick auf Formgebungseigenschaften für Haarfasern. Bei diesem Modell zur Ermittlung der Haarfixierung werden die prozentualen Veränderungen von Ausgangs- zu Endlänge von definiert präparierten Locken im Vergleich zu Haartressenlänge aufgezeichnet. Die Curl Retention Eigenschaften von Haarpflege- und Stylingprodukten bei hoher Luftfeuchtigkeit werden bei 23°C und 90% relativer Luftfeuchtigkeit über einen Zeitraum von sieben Stunden verfolgt.

Es werden aus braunem europäischem Haar 15 cm lange Haartressen als Bündel à 3,5 g zusammengestellt, mit einem Bindfaden gebunden und mit einem geeigneten Klebstoff dauerhaft fixiert. Die Haartressen werden jeweils zweimal mit 3 ml einer Ammonium Lauryl Sulfat Lösung (25%ig), STEPANOL(R) ALS 25, Fa. STEPAN Company und anschließend 30°C warmen, vollentsalztem Wasser gewaschen. Die Haartressen werden gekämmt und getrocknet. Die trockenen Tressen werden mit 20 Hüben eines 3% aktiven Pump Sprays besprüht, einmal durchgekämmt und dann auf einen Kunststoffstab mit einem Durchmesser von 1,4cm gewickelt, mit einer Hülle temporär fixiert und bei 50°C über Nacht getrocknet.

Die Locken werden von den Kunststoffstäben vorsichtig abgestreift. Nach kurzem Abkühlen werden die Locken an einer skalierten Hängevorrichtung in einem Klimaschrank bei 23°C und 90% relativer Luftfeuchtigkeit befestigt. Zuvor wurde die Ausganglänge der Locke bestimmt und notiert. In bestimmten Zeitintervallen werden die Lockenlängen, das heißt die Veränderung zur Ausgangslänge über einen Zeitraum von 7 h abgelesen.

Die Berechnung erfolgt nach Gleichung (G-I),

$$\% \ CURL \ RETENTION \ = \ \frac{L - L_t}{L - L_0} \ x \ 100 \qquad (\text{G-I})$$

wobei

L = Länge der Haartresse
$L_o$ = Ausgangslänge der Locke
$L_t$ = Länge der Locke nach/während der Messung

bedeuten.

Je höher der Prozentwert für die Curl Retention nach einer Zeit t ist, desto besser sind die Formgebungseigenschaften bzw. Fixierungseigenschaften der kosmetischen Formulierung. Die Locken hängen sich weniger stark aus.

**Beispiele für kosmetische Zusammensetzungen:**

Vorvernetzte Emulsionen sind bevorzugt Emulsionen, die nach Entfernen von Wasser elastische Filme bilden und in rheologischen Messungen einen tan δ < 1 aufweisen.

**Beispiele A1-a, A1-c und A2-a** (Rinse-Off Conditioner (Spülung)) Die nachfolgenden Beispiele repräsentieren erfindungsgemäße kosmetische Zusammensetzungen **A1-a, A1-c** und **A2-a** gemäß Tabelle 2 enthaltend die vorvernetzten Emulsionen **B1-a, B1-c** und **B2** aus den entsprechenden Beispielen. Der Aktivgehalt an Organopolysiloxanen in den kosmetischen Zusammensetzungen beträgt 0,5%.

Herstellanweisung:

Wasser wird vorgelegt und unter Rühren auf 75°C erwärmt. Dabei werden 2,0 Teile Hydroxyethylcellulose zugegeben. Wenn 65°C erreicht sind, werden 0,5 Teile Polysorbate 80, 0,5 Teile Stearyl Alcohol, 0,5 Teile Cetyl Alcohol und 0,2 Teile Behentrimonium Chloride zugegeben. Die Mischung wird gerührt bis 75°C erreicht sind und die Inhaltsstoffe gelöst vorliegen. Dann wird die Mischung abgekühlt. Während des Abkühlens werden 0,1 Teile Citric Acid und 0,2 Teile Tetrasodium EDTA zugegeben. Bei 35°C werden 0,9 Teile Phenoxyethanol, Ethylhexylglycerin zugegeben. Unter weiterem Rühren erfolgt die Zugabe der Emulsion aus den Beispielen. Die Zusammensetzung wird 15 Minuten unter Rühren homogenisiert.

**Tabelle 2:** Rinse-Off Conditioner **A1-a, A1-c** und **A2-a**

| Bestandteile (INCI-name) | Bsp. **A1-a** [Gew.-teile] | Bsp. **A1-c** [Gew.-teile] | Bsp. **A2-a** [Gew.-teile] |
|---|---|---|---|
| Water | ad 100 | ad 100 | ad 100 |
| Hydroxyethylcellulose[1] | 2,0 | 2,0 | 2,0 |
| Cetyl Alcohol [2] | 0,5 | 0,5 | 0,5 |
| Polysorbate 80 [3] | 0,5 | 0,5 | 0,5 |
| Behentrimonium Chloride [4] | 0,2 | 0,2 | 0,2 |
| Stearyl Alcohol [5] | 0,5 | 0,5 | 0,5 |
| Citric Acid [6] | 0,1 | 0,1 | 0,1 |
| Tetrasodium EDTA [7] | 0,2 | 0,2 | 0,2 |
| Emulsion **B1-a** aus Beispiel 1 | 1,43 | | |
| Emulsion **B1-c** aus Beispiel 1 | | 1,43 | |
| Emulsion **B2** aus Beispiel 2 | | | 1,16 |
| Phenoxyethanol, Ethylhexylglycerin [8] | 0,9 | 0,9 | 0,9 |
| Die in Tabelle 2 genannten Rohstoffe sind unter folgenden Handelsnamen erhältlich: 1)Hydroxyethylcellulose: Natrosol 250 HR, Ashland 2)Cetylalkohol: Cetylalkohol, Merck KGaA 3)Polysorbate 80: Tween™ 80, Croda GmbH 4) Behentrimonium Chloride: Genamin® KDMP, Clariant GmbH 5)Stearylalkohol: Stearylalkohol Merck KGaA 6)Citric Acid: Citric Acid, Sigma 7) Tetrasodium EDTA: EDETA® B Pulver, BASF Corporation 8)Phenoxyethanol, Ethylhexylglycerin: Euxyl PE 9010, Schülke & Mayr | | | |

**Vergleichsversuch VA1 und VA2** (Rinse-Off-Conditioner (Spülung)) Die nachfolgenden Vergleichsversuche **VA1 und VA2** repräsentieren nicht-erfindungsgemäße kosmetische Zusammensetzungen enthaltend die nicht-vorvernetzten wässrigen Dispersionen **E1** und **E2** aus den entsprechenden Beispielen. Der Aktivgehalt an Organopolysiloxanen in den kosmetischen Zusammensetzungen beträgt 0,5%.

Bei der Herstellung der kosmetischen Zusammensetzungen der Vergleichsversuche **VA1** und **VA2** wurde die Arbeitsweise der Beispiele **A1-a, A1-c** und **A2-a** wiederholt, mit der Abänderung, dass anstelle der Emulsionen **B1-a, B1-**

**c** und **B2** (Emulsionen von erfindungsgemäßen vorvernetzten Organopolysiloxanen) die unvernetzten Emulsionen **E1** und **E2** eingesetzt werden.

Herstellanweisung:

Wasser wird vorgelegt und unter Rühren auf 75°C erwärmt. Dabei werden 2,0 Teile Hydroxyethylcellulose zugegeben. Wenn 65°C erreicht sind, werden 0,5 Teile Polysorbate 80, 0,5 Teile Stearyl Alcohol, 0,5 Teile Cetyl Alcohol und 0,2 Teile Behentrimonium Chloride zugegeben. Die Mischung wird gerührt bis 75°C erreicht sind und die Inhaltsstoffe gelöst vorliegen. Dann wird die Mischung abgekühlt. Während des Abkühlens werden 0,1 Teile Citric Acid und 0,2 Teile Tetrasodium EDTA zugegeben. Bei 35°C werden 0,9 Teile Phenoxyethanol, Ethylhexylglycerin zugegeben. Unter weiterem Rühren erfolgt die Zugabe der Emulsion gemäß Tabelle 3 für die Vergleichsversuche. Die Zusammensetzung wird 15 Minuten unter Rühren homogenisiert.

**Tabelle 3:** Rinse-Off Conditioner **VA1** und **VA2**

| Bestandteile (INCI-name) | Vgl.-versuch **VA1** [Gew.-teile] | Vgl.-versuch **VA2** [Gew.-teile] |
|---|---|---|
| Water | ad 100 | ad 100 |
| Hydroxyethyl-cellulose [1] | 2,0 | 2,0 |
| Cetyl Alcohol [2] | 0,5 | 0,5 |
| Polysorbate 80 [3] | 0,5 | 0,5 |
| Behentrimonium Chloride [4] | 0,2 | 0,2 |
| Stearyl Alcohol [5] | 0,5 | 0,5 |
| Citric Acid [6] | 0,1 | 0,1 |
| Tetrasodium EDTA [7] | 0,2 | 0,2 |
| Emulsion **E1** | 1,43 | |
| Emulsion **E2** | | 1,16 |
| Phenoxyethanol, Ethylhexylglycerin[8] | 0,9 | 0,9 |
| Die in Tabelle 3 genannten Rohstoffe sind unter folgenden Handelsnamen erhältlich:<br>1)Hydroxyethylcellulose: Natrosol 250 HR, Ashland<br>2)Cetylalkohol: Cetylalkohol, Merck KGaA<br>3)Polysorbate 80: Tween™ 80, Croda GmbH<br>4) Behentrimonium Chloride: Genamin® KDMP, Clariant GmbH<br>5)Stearylalkohol: Stearylalkohol Merck KGaA<br>6)Citric Acid: Citric Acid, Sigma<br>7) Tetrasodium EDTA: EDETA® B Pulver, BASF Corporation<br>8)Phenoxyethanol, Ethylhexylglycerin: Euxyl PE 9010, Schülke & Mayr | | |

**Vergleichsversuch VA1-DEO** (Rinse-Off-Conditioner (Spülung)) Der nachfolgende Vergleichsversuch **VA1-DEO** repräsentiert eine nicht-erfindungsgemäße kosmetische Zusammensetzung enthaltend eine wässrige Dispersion eines vorvernetzten Organopolysiloxans. Der Aktivgehalt an Organopolysiloxanen in der kosmetischen Zusammensetzung beträgt 0,5%.

Bei der Herstellung der kosmetischen Zusammensetzung des Vergleichsversuches **VA1-DEO** wurde die Arbeitsweise der Beispiele **VA1 und VA2** wiederholt, mit der Abänderung, dass anstelle der Emulsionen **E1** und **E2** (Emulsionen von nicht-vorvernetzten Organopolysiloxanen) die vorvernetzte, nicht-erfindungsgemäße Emulsion **V1** eingesetzt wird.

Herstellanweisung:

Wasser wird vorgelegt und unter Rühren auf 75°C erwärmt. Dabei werden 2,0 Teile Hydroxyethylcellulose zugegeben. Wenn 65°C erreicht sind, werden 0,5 Teile Polysorbate 80, 0,5 Teile Stearyl Alcohol, 0,5 Teile Cetyl Alcohol und 0,2 Teile Behentrimonium Chloride zugegeben. Die Mischung wird gerührt bis 75°C erreicht sind und die Inhaltsstoffe gelöst vorliegen. Dann wird die Mischung abgekühlt. Während des Abkühlens werden 0,1 Teile Citric Acid und 0,2 Teile Tetrasodium EDTA zugegeben. Bei 35°C werden 0,9 Teile Phenoxyethanol, Ethylhexylglycerin zugegeben. Unter weiterem Rühren erfolgt die Zugabe der Emulsion gemäß Tabelle 4 für den Vergleichsversuch. Die Zusammensetzung wird 15 Minuten unter Rühren homogenisiert.

**Tabelle 4:** Rinse-Off Conditioner **VA1-DEO**

| Bestandteile (INCI-name) | Vgl.-versuch **VA1-DEO** [Gew.-teile] |
|---|---|
| Water | ad 100 |
| Hydroxyethyl-cellulose [1] | 2,0 |
| Cetyl Alcohol [2] | 0,5 |
| Polysorbate 80 [3] | 0,5 |
| Behentrimonium Chloride [4] | 0,2 |
| Stearyl Alcohol [5] | 0,5 |
| Citric Acid [6] | 0,1 |
| Tetrasodium EDTA [7] | 0,2 |
| Emulsion **V1** | 1,43 |
| Phenoxyethanol, Ethylhexylglycerin[8] | 0,9 |

Die in Tabelle 4 genannten Rohstoffe sind unter folgenden Handelsnamen erhältlich:
1)Hydroxyethylcellulose: Natrosol 250 HR, Ashland
2)Cetylalkohol: Cetylalkohol, Merck KGaA
3)Polysorbate 80: Tween™ 80, Croda GmbH
4) Behentrimonium Chloride: Genamin® KDMP, Clariant GmbH
5)Stearylalkohol: Stearylalkohol Merck KGaA
6)Citric Acid: Citric Acid, Sigma
7) Tetrasodium EDTA: EDETA® B Pulver, BASF Corporation
8)Phenoxyethanol, Ethylhexylglycerin: Euxyl PE 9010, Schülke & Mayr

**Vergleich der Rinse-Off Conditioner der erfindungsgemäßen Beispiele A1-a, A1-c und A2-a mit den Vergleichsversuchen VA1 und VA2**
Die angeführten Beispiele und Vergleichsversuche unterscheiden sich dadurch, dass im Fall der Beispiele **A1-a, A1-c** und **A2-a** erfindungsgemäße wässrige Dispersionen von vorvernetzten Organopolysiloxanen Einsatz fanden, in den Vergleichsversuchen **VA1** und **VA2** jeweils analoge, wässrige Dispersionen der entsprechenden nicht vorvernetzten Organopolysiloxane. Der Aktivgehalt an Organopolysiloxanen in den kosmetischen Zusammensetzungen beträgt 0,5%.
Im direkten Vergleich korrelieren folgende Beispiele / Vergleichsversuche:

Beispiel **A1-a** - Vergleichsversuch **VA1**
Beispiel **A1-c** - Vergleichsversuch **VA1**
Beispiel **A2-a** - Vergleichsversuch **VA2**

**Nasskämmkraft nach Behandlung von geschädigtem Haar mit Rinse-Off Conditioner und nach Simulierung mehrerer Shampoowäschen durch Rühren der Haare in einer wässrigen Tensidlösung (Persistenzeffekt)**

Beispiele **A1-a** und **A1-c** - Vergleichsversuch **VA1**

Der Einsatz der wässrigen Dispersion **B1-a und B1-c** von erfindungsgemäß vorvernetzten Organopolysiloxanen im Rinse-Off Conditioner (Beispiele **A1-a und A1-c)** führt zu einer Verbesserung von konditionierenden Eigenschaften wie z.B. der Verringerung der Kämmkräfte im nassen Zustand im Vergleich zum nicht erfindungsgemäßen Rinse-Off Conditioner aus Beispiel **VA1,** der die Dispersion eines nicht vorvernetzten Organopolysiloxans **E1** enthält. Ziel der erfindungsgemäßen Rinse-Off Conditioner ist zusätzlich, dass die pflegenden Eigenschaften auch nach mehreren Shampoowäschen der Haare erhalten bleiben. Der Prozess der Shampoowäsche wird in diesem Beispiel durch das vierstündige Rühren von Rinse-Off-Conditioner behandelten Haarbündeln in einer Tensidlösung simuliert. Die Details dieser Behandlung sind oben unter *Testmethoden* beschrieben.
Die Ergebnisse der Bestimmung der Kämmkraft im nassen Zustand sind nachfolgend für die Rinse-Off Conditioner der Beispiele **A1-a, A1-c** und **VA1** in Tabelle 5 aufgeführt.

**Tabelle 5:** Rinse-Off Conditioner / Ergebnisse der Nasskämmkraftreduktion auf geschädigtem kaukasischem Haar nach Behandlung mit einem erfindungsgemäßen Rinse-Off Conditioner im Vergleich zu einem nicht-erfindungsgemäßen Conditioner und nach Simulierung mehrerer Shampoowäschen durch vierstündiges Rühren der Haare in einer wässrigen Tensidlösung. Alle Ergebnisse beziehen sich auf den Vergleich zu unbehandelten Haartressen.

| Bsp./ Vgl. | Reduktion Nasskämmkraft nach Conditionerbehandlung [%] | Reduktion Nasskämmkraft nach Conditionerbehandlung und anschließendem Rühren der Haare in einer wässrigen Tensidlösung (Persistenztest) [%] |
|---|---|---|
| A1-a | 84 | 39 |
| A1-c | 81 | 31 |
| VA1 | 79 | 21 |

Durch Behandlung mit dem erfindungsgemäßen Rinse-Off Conditioner enthaltend eine wässrige Emulsion eines vorvernetzten Organopolysiloxans **B1-a** (Beispiel **A1-a**) kann mit 84% eine signifikante Reduktion der Nasskämmkraft der Haartressen gemessen werden. Insbesondere bleibt eine hohe konditionierende Wirkung nach Rühren der behandelten Haare in einer Tensidlösung erhalten, die sich in einer verbleibenden Nasskämmkraftreduktion von 39% widerspiegelt. Analog wird durch eine Behandlung mit einem erfindungsgemäßen Rinse-Off Conditioner enthaltend die wässrige Emulsion eines vorvernetzten Organopolysiloxans **B1-c** (Beispiel **A1-c**) eine hohe Reduktion der Nasskämmkraft der Haartressen von 81% gemessen. Nach Rühren der behandelten Haare in Tensidlösung verbleibt eine Nasskämmkraftreduktion von 31%.

Haare behandelt mit einem nicht erfindungsgemäßen Rinse-Off Conditioner enthaltend die Emulsion **E1** (Vergleichsbeispiel **VA1**) weisen eine leicht verringerte Nasskämmkraftreduktion von 79% auf. Nach der Tensidbehandlung erhält man jedoch eine im Vergleich zu Beispiel **A-1a und A-1c** deutlich geringere Nasskämmkraftreduktion von 21%. Die Behandlung der Haare in Tensidlösung stellt eine Simulation mehrerer Haarwäschen mit Shampoo dar und demonstriert, dass die erfindungsgemäßen Rinse-Off Conditioner eine bessere Waschbeständigkeit zeigen und die konditionierenden Eigenschaften länger erhalten bleiben als bei einer Haarbehandlung mit einem nicht erfindungsgemäßen Rinse-Off Conditioner.

**Vergleich der Silicondeponierung nach Behandlung von geschädigtem Haar mit Rinse-Off Conditioner und nach Simulierung mehrerer Shampoowäschen durch vierstündiges Rühren der Haare in einer wässrigen Tensidlösung**

Beispiel **A2-a** - Vergleichsversuch **VA2**

Als weiteres Kriterium wurde die Effizienz der Siliconabscheidung auf geschädigtem Haar nach Behandlung mit Rinse-Off Conditionern bewertet und zusätzlich untersucht, wieviel des konditionierend wirkenden Organopolysiloxans auf der Haaroberfläche nach Simulation von Shampoowäschen durch vierstündiges Rühren der Haare in einer Tensidlösung verbleibt. Die Ergebnisse sind in Tabelle 6 zusammengefasst. Die Bestimmung der Silicondeponierung ist oben unter *Testmethoden* beschrieben.

**Tabelle 6:** Rinse-Off Conditioner / Silicondeponierung

| Bsp./ Vgl. | Silicondeponierung [ppm] | Silicondeponierung nach Rühren der Haare in einer wässrigen Tensidlösung (Persistenztest) [ppm] |
|---|---|---|
| A2-a | 55 | 34 |
| VA2 | 36 | 21 |

Tabelle 6 zeigt, dass die Deponierung des Silicons - auch nach Shampoowäschen - bei dem erfindungsgemäßen Beispiel **A2-a** sowohl effektiver als auch beständiger gegenüber Shampoowäschen verglichen mit dem nicht-erfindungsgemäßen Vergleichsversuch **VA2** ist.

**Vergleich der Rinse-Off Conditioner des erfindungsgemäßen Beispiels A1-a mit dem Vergleichsversuch VA1-DEO**

Das angeführte Beispiel und der Vergleichsversuch unterscheiden sich dadurch, dass im Fall des Beispiels **A1-a** eine erfindungsgemäße wässrige Dispersion von vorvernetzten Organopolysiloxanen Einsatz fand, im Vergleichsversuch **VA1-DEO** eine analoge, nicht-erfindungsgemäße wässrige Dispersionen der entsprechenden vorvernetzten Organopolysiloxane. Der Aktivgehalt an Organopolysiloxanen in den kosmetischen Zusammensetzungen beträgt 0,5%.

**Nasskämmkraft nach Behandlung von geschädigtem Haar mit Rinse-Off Conditioner und nach Simulierung mehrerer Shampoowäschen durch Rühren der Haare in einer wässrigen Tensidlösung (Persistenzeffekt)**

Beispiel **A1-a** - Vergleichsversuch **VA1-DEO**

Der Einsatz der wässrigen Dispersion **B1-a** von erfindungsgemäß vorvernetzten Organopolysiloxanen im Rinse-Off Conditioner (Beispiel **A1-a**) führt zu einer Verbesserung von konditionierenden Eigenschaften wie z.B. der Verringerung der Kämmkräfte im nassen Zustand im Vergleich zum nicht erfindungsgemäßen Rinse-Off Conditioner aus Beispiel **VA1-DEO,** der die Dispersion eines vorvernetzten Organopolysiloxans **(Vergleichsversuch V1)** enthält. Ziel des erfindungsgemäßen Rinse-Off Conditioners ist zusätzlich, dass die pflegenden Eigenschaften auch nach mehreren Shampoowäschen der Haare erhalten bleiben. Der Prozess der Shampoowäsche wird in diesem Beispiel durch das vierstündige Rühren von Rinse-Off-Conditioner behandelten Haarbündeln in einer Tensidlösung simuliert. Die Details dieser Behandlung sind oben unter *Testmethoden* beschrieben.

Die Ergebnisse der Bestimmung der Kämmkraft im nassen Zustand sind nachfolgend für die Rinse-Off Conditioner der Beispiele **A1-a** und **VA1-DEO** in Tabelle 7 aufgeführt.

**Tabelle 7:** Rinse-Off Conditioner / Ergebnisse der Nasskämmkraftreduktion auf geschädigtem kaukasischem Haar nach Behandlung mit einem erfindungsgemäßen Rinse-Off Conditioner im Vergleich zu einem nicht-erfindungsgemäßen Conditioner und nach Simulierung mehrerer Shampoowäschen durch vierstündiges Rühren der Haare in einer wässrigen Tensidlösung. Alle Ergebnisse beziehen sich auf den Vergleich zu unbehandelten Haartressen.

| Bsp./ Vgl. | Reduktion Nasskämmkraft nach Conditionerbehandlung [%] | Reduktion Nasskämmkraft nach Conditionerbehandlung und anschließendem Rühren der Haare in einer wässrigen Tensidlösung (Persistenztest) [%] |
|---|---|---|
| **A1-a** | 84 | 39 |
| **VA1-DEO** | 77 | 20 |

Durch Behandlung mit dem erfindungsgemäßen Rinse-Off Conditioner enthaltend eine wässrige Emulsion eines vorvernetzten Organopolysiloxans **B1-a** (Beispiel **A1-a**) kann mit 84% eine signifikante Reduktion der Nasskämmkraft der Haartressen gemessen werden. Insbesondere bleibt eine hohe konditionierende Wirkung nach Rühren der behandelten Haare in einer Tensidlösung erhalten, die sich in einer verbleibenden Nasskämmkraftreduktion von 39% widerspiegelt.

Haare behandelt mit einem nicht erfindungsgemäßen Rinse-Off Conditioner **VA1-DEO** enthaltend die Emulsion aus **Vergleichsbeispiel V1** weisen eine leicht verringerte Nasskämmkraftreduktion von 77% auf. Nach der Tensidbehandlung erhält man jedoch eine im Vergleich zu Beispiel **A-1a** deutlich geringere Nasskämmkraftreduktion von 20%. Die Behandlung der Haare in Tensidlösung stellt eine Simulation mehrerer Haarwäschen mit Shampoo dar und demonstriert, dass der erfindungsgemäße Rinse-Off Conditioner eine bessere Waschbeständigkeit zeigt und die konditionierenden Eigenschaften länger erhalten bleiben als bei einer Haarbehandlung mit einem nicht erfindungsgemäßen Rinse-Off Conditioner.

**Beispiel A3-a**

Kosmetische Zusammensetzung: Shampoo

Das nachfolgende Beispiel repräsentiert eine erfindungsgemäße kosmetische Zusammensetzung **A3-a** enthaltend die Emulsion **B3.** Das Vergleichsbeispiel **VA3** repräsentiert eine marktübliche Shampooformulierung enthaltend eine Emulsion eines Dimethicons (Dimethylpolysiloxans; BELSIL® DM 5102 E (erhältlich bei der Wacker Chemie AG) mit einer Viskosität von 60.000 mm$^2$/s (bei 25°C). Der Aktivgehalt an Organopolysiloxanen in den kosmetischen Zusammensetzungen beträgt 1,3%.

Die Zusammensetzung der Shampoos ist in der Tabelle 8 zusammengefasst.

**Herstellanweisung:**

32,11 Teile Wasser werden vorgelegt und unter Rühren auf 50°C erwärmt. Dabei werden 0,20 Teile Guar Hydroxylpropyltrimonium Chloride, 6,06 Teile Sodium Lauryl Sulfate, 29,90 Teile Sodium Laureth Sulfate, 0,05 Teile Citric Acid und 5,0 Teile Cocamidopropyl Betaine zugegeben. Die Mischung wird gerührt bis 50°C erreicht sind und die Inhaltsstoffe gelöst vorliegen. Dann wird die Mischung abgekühlt. In einem separaten Gefäß werden 20,0 Teile Wasser vorgelegt, unter Rühren 0,60 Teile Carbomer zugegeben und gerührt bis eine homogene Mischung entsteht. Nun werden 0,06 Teile Lactic Acid zugegeben. Diese Mischung wird zur ersten Mischung zugegeben. Bei 40°C werden 0,95 Teile Phenoxyethanol, Ethylhexylglycerin zugegeben. Unter weiterem Rühren erfolgt die Zugabe von 0,30 Teilen C12-13 Alkyl Lactate, 2,91 Teilen der erfindungsgemäßen Emulsion **B3** bzw. 2,60 Teilen der nicht-erfindungsgemäßen Dimethicon-Emulsion BELSIL® DM 5102 E, 0,40 Teilen Sodium Hydroxide und 0,66 Teilen Sodium Chloride. Der erforderliche pH-Wert von 6,5 ist bei Bedarf durch Zugabe von Sodium Hydroxide einzustellen.

**Tabelle 8:** Shampooformulierung **A3-a** und **VA3**

| Bestandteile (INCI-Name) | Beispiel **A3-a** [Gew.-teile] | Vergleichsversuch **VA3** [Gew.-teile] |
|---|---|---|
| Citric Acid[1] | 0,05 | 0,05 |
| Cocamidopropyl Betaine[2] | 5,00 | 5,00 |
| Sodium Laureth Sulfate[3] | 29,90 | 29,90 |
| Guar Hydroxypropyltrimonium Chloride[4] | 0,20 | 0,20 |
| Sodium Lauryl Sulfate[5] | 6,06 | 6,06 |
| Aqua (DI Water) | 32,11 | 32,11 |
| Carbomer[6] | 0,60 | 0,60 |
| Lactic Acid[7] | 0,06 | 0,06 |
| Aqua (DI Water) | 20,00 | 20,00 |
| Phenoxyethanol, Ethylhexylglycerin[8] | 0,95 | 0,95 |
| C12-13 Alkyl Lactate[9] | 0,30 | 0,30 |
| Emulsion **B3** aus Beispiel 3 | 2,91 | |
| BELSIL® DM 5102 E[10] (Dimethicon-Emulsion) | | 2,60 |
| Sodium Hydroxide[11] | 0,40 | 0,40 |
| Sodium Chloride[12] | 0,66 | 0,66 |

1) Citric Acid: Citric Acid, Sigma
2) Cocamidopropyl Betaine: Genagen CAB 818 30%, Clariant
3) Sodium Laureth Sulfat: Genapol® LRO 26,5%, Clariant
3) Glycol Distearate: Genapol® PMS, Clariant GmbH
4) Guar Hydroxypropyltrimonium Chloride: N-Hance® BF 13, Ashland
5) Sodium Lauryl Sulfat: Texapon K 12 G, BASF
6) Carbomer: Carbopol 980, Lubrizol
7) Lactic acid: L-(+)Milchsäure, 90%, Bernd Kraft GmbH
8) Phenoxyethanol, Ethylhexylglycerin: Euxyl PE 9010, Schülke & Mayr
9) C12-13 Alkyl Lactate: Ceraphyl™ 41 ester, Ashland
10) BELSIL® DM 5102 E, erhältlich bei der Wacker Chemie AG
11) Sodium Hydroxide: Natriumhydroxid, Sigma-Aldrich
12) Sodium Chloride: Natriumchlorid reinst, Bernd Kraft GmbH

**Vergleich der Shampoos des erfindungsgemäßen Beispiels A3-a mit dem Vergleichsversuch VA3**

Das angeführte Beispiel und der Vergleichsversuch unterscheiden sich dadurch, dass im Fall des Beispiels **A-3a** eine wässrige Dispersion von vorvernetzten Organopolysiloxanen Einsatz fand. Im Vergleichsversuch **VA3** wird die handelsübliche, wässrige Dimethylpolysiloxan-Emulsion BELSIL® DM 5102 E (Wacker Chemie AG) eingesetzt, die als konditionierenden Wirkstoff ein Dimethicon (Dimethylpolysiloxan) mit der Viskosität von 60.000 $mm^2$/s enthält. Der

Aktivgehalt an Organopolysiloxanen in den kosmetischen Zusammensetzungen beträgt 1,3%.

Die Ergebnisse der kosmetischen Wirkung der Shampoos sind in der Tabelle 9 zusammengefasst.

**Tabelle 9:** Shampoo / Ergebnisse der Nasskämmkraftreduktion und Verbesserung der Weichheit auf geschädigtem Haar nach Behandlung mit Shampoo. Alle Ergebnisse im Vergleich zu unbehandelten Haartressen.

| Bsp. /Vgl. | Reduktion Nasskämmkraft [%] | Verbesserung Weichheit [%] |
|---|---|---|
| Bsp. A3-a | 50 | 25 |
| Vergleichsversuch VA3*) | 20 | 2 |
| *) BELSIL® DM 5102 E (Dimethicon-Emulsion), erhältlich bei der Wacker Chemie AG | | |

Die Shampoos gemäß des Beispiels **A-3a** mit der erfindungsgemäßen Emulsion zeigen eine wesentlich höhere Reduktion der Nasskämmkraft und eine deutlich verbesserte Weichheit (gemäß Zug-Prüfung) im Vergleich zu dem Shampoo gemäß Vergleichsversuch **VA3** mit der handelsüblichen Dimethicon-Emulsion.

**Beispiel A3-b:**

Kosmetische Zusammensetzung - Shampoo

Das nachfolgende Beispiel repräsentiert eine kosmetische Zusammensetzung enthaltend die Emulsion **B3.** Der Aktivgehalt an Organopolysiloxanen in der kosmetischen Zusammensetzung beträgt 1,0%.

Die Zusammensetzung des Shampoos ist in der Tabelle 10 zusammengefasst.

Herstellanweisung:

0,30 Teile Guar Hydroxypropyltrimonium Chloride werden in Wasser dispergiert. 41,50 Teile Sodium Laureth Sulfate werden langsam eingerührt und die Mischung wird schrittweise auf 75°C erwärmt. Während des Erwärmens werden bei 50°C 0,20 Teile PEG-150 Distearate zugegeben, bei 65°C erfolgt die Zugabe von 0,50 Teilen Glycol Distearate. Anschließend wird die Mischung abgekühlt. Bei Erreichen von 35°C werden 0,90 Teile Phenoxyethanol, Ethylhexylglycerin und die Emulsion entsprechend dem Beispiel zugegeben und 5 Minuten gerührt. Abschließend werden 13,4 Teile Cocamidopropyl Betaine zugegeben und weitere 10 Minuten gerührt.

Geschädigte Haare, die mit dem Shampoo aus Beispiel **A3-b** behandelt wurden, sind weicher gemäß Panel-Test als geschädigte, unbehandelte Haare.

**Tabelle 10:** Shampooformulierungen **A3-b** (Angabe in Gew.-teilen)

| Bestandteile (INCI-name) | Bsp. A3-b |
|---|---|
| Aqua (Water VES) | ad 100 |
| Guar Hydroxypropyltrimonium Chloride [1] | 0,30 |
| Sodium Laureth Sulfate [2] | 41,50 |
| Glycol Distearate [3] | 0,50 |
| PEG-120 Methyl Glucose Dioleate [4] | 0,20 |
| Emulsion **B3** aus Beispiel 3 | 2,27 |
| Cocamidopropyl Betaine [6] | 13,33 |
| Phenoxyethanol, Ethylhexylglycerin [7] | 0, 90 |
| 1) Guar Hydroxypropyltrimonium Chloride: N-Hance® 3196, Ashland. 2) Sodium Laureth Sulfat: Genapol® LRO 26,5%, Clariant GmbH 3) Glycol Distearate: HALLSTAR® EGDS, The Hallstar Company 4) PEG-120 Methyl Glucose Dioleate: Glucamate™ DOE 120 thickener, Lubrizol 5) BELSIL® DM 5102 E, erhältlich bei der Wacker Chemie AG 6) Cocamidopropyl Betaine: Genagen® CAB 30%, Clariant GmbH 7) Phenoxyethanol, Ethylhexylglycerin: Euxyl PE 9010, Schülke & Mayr | |

EP 4 504 821 B1

**Beispiel A3-c:**

Kosmetische Zusammensetzung - Shampoo

Das nachfolgende Beispiel repräsentiert eine kosmetische Zusammensetzung enthaltend die Emulsion **B3.** Der Aktivgehalt an Organopolysiloxanen in der kosmetischen Zusammensetzung beträgt 1,0%

Die Zusammensetzung des Shampoos ist in der Tabelle 11 zusammengefasst.

**Tabelle 11:** Shampooformulierung **A3-c** (Angabe in Gew.-teilen)

| Bestandteile (INCI-name) | Bsp. A3-c |
|---|---|
| Aqua (Water VES) | ad 100 |
| Polyquaternium-10 [1] | 0, 10 |
| Sodium Laureth Sulfate [2] | 52,80 |
| PEG-150 Distearate [3] | 0,25 |
| Cocamide MEA [4] | 1,00 |
| Emulsion **B3** aus Beispiel 3 | 2,27 |
| Cocamidopropyl Betaine [5] | 10,06 |
| Phenoxyethanol, Ethylhexylglycerin [6] | 0,95 |
| 1) Polyquaternium-10: UCARE Polymer JR 400, Dow Chemical. <br> 2) Sodium Laureth Sulfat: Genapol® LRO 26,5%, Clariant GmbH <br> 3) PEG-150 Distearate: Eumulgin® EO 33, BASF AG <br> 4) Cocamide MEA: Comperlan 100, BASF AG <br> 5) Cocamidopropyl Betaine: Genagen® CAB 30%, Clariant GmbH <br> 7) Phenoxyethanol, Ethylhexylglycerin: Euxyl PE 9010, Schülke & Mayr | |

Geschädigte Haare, die mit dem Shampoo aus Beispiel **A3-c** behandelt wurden, sind weicher gemäß Panel-Test als geschädigte, unbehandelte Haare.

**Beispiele A1-b und A2-b**

Rinse-Off Conditioner

Die nachfolgenden Beispiele repräsentieren kosmetische Zusammensetzungen **A1-b** und **A2-b** enthaltend die Emulsionen **B-1b** und **B2.** Der Aktivgehalt an Organopolysiloxanen in den kosmetischen Zusammensetzungen beträgt 2,0%

Die Zusammensetzung der Rinse-Off Conditioner ist in der Tabelle 12 zusammengefasst.

Herstellanweisung:

Wasser wird vorgelegt und unter Rühren auf 75°C erwärmt. Dabei werden 1,1 Teile Hydroxyethylcellulose zugegeben. Wenn 65°C erreicht sind, werden 0,5 Teile Stearamidopropyl Dimethylamine, 1,0 Teile Polysorbate 80, 3,0 Teile Stearyl Alcohol, 2,0 Teile Cetyl Alcohol und 1,8 Teile Behentrimonium Chloride zugegeben. Die Mischung wird gerührt bis 75°C erreicht sind und die Inhaltsstoffe gelöst vorliegen. Dann wird die Mischung abgekühlt. Während des Abkühlens werden 0,2 Teile Citric Acid und 0,2 Teile Tetrasodium EDTA zugegeben. Bei 35°C werden 0,9 Teile Phenoxyethanol, Ethylhexylglycerin zugegeben. Unter weiterem Rühren erfolgt die Zugabe der Emulsionen aus den Beispielen. Die Zusammensetzung wird 15 Minuten unter Rühren homogenisiert.

**Tabelle 12:** Rinse-Off Conditioner **A1-b und A2-b**

| Bestandteile (INCI-name) | Beispiel **A1-b** [Gew.-teile] | Beispiel **A2-b** [Gew.-teile] |
|---|---|---|
| Water | ad 100 | ad 100 |
| Hydroxyethylcellulose [1] | 1,1 | 1,1 |

(fortgesetzt)

| Bestandteile (INCI-name) | Beispiel **A1-b** [Gew.-teile] | Beispiel **A2-b** [Gew.-teile] |
|---|---|---|
| Cetyl Alcohol [2] | 2, 0 | 2, 0 |
| Polysorbate 80 [3] | 1, 0 | 1, 0 |
| Behentrimonium Chloride [4] | 1, 8 | 1, 8 |
| Stearamidopropyl Dimethylamine [5] | 0,5 | 0,5 |
| Stearyl Alcohol [6] | 3, 0 | 3, 0 |
| Citric Acid [7] | 0,2 | 0,2 |
| Tetrasodium EDTA [8] | 0,2 | 0,2 |
| Emulsion **B1-b** aus Beispiel 1 | 5,71 | |
| Emulsion **B2** aus Beispiel 2 | | 4, 64 |
| Phenoxyethanol, Ethylhexylglycerin [9] | 0, 9 | 0, 9 |
| Die in Tabelle 12 genannten Rohstoffe sind unter folgenden Handelsnamen erhältlich:<br>1)Hydroxyethylcellulose: Natrosol 250 HR, Ashland<br>2)Cetylalkohol: Cetylalkohol, Merck KGaA<br>3)Polysorbate 80: Tween™ 80, Croda GmbH<br>4) Behentrimonium Chloride: Genamin® KDMP, Clariant GmbH<br>5)Stearamidopropyl Dimethylamine, Incromine™ SB, Croda GmbH<br>6)Stearylalkohol: Stearylalkohol, Merck KGaA<br>7)Citric Acid: Citric Acid, Sigma<br>8) Tetrasodium EDTA: EDETA® B Pulver, BASF Corporation<br>9)Phenoxyethanol, Ethylhexylglycerin: Euxyl PE 9010, Schülke & Mayr | | |

Geschädigte Haare, die mit dem Rinse-Off Conditionern aus den Beispielen **A1-b und A2-b** behandelt wurden, sind weicher gemäß Panel-Test als geschädigte, unbehandelte Haare.

**Beispiel A4**

Kosmetische Zusammensetzung: Pflegendes Styling Spray

Das nachfolgende Beispiel repräsentiert eine erfindungsgemäße kosmetische Zusammensetzung **A4** enthaltend die Emulsion **B1-a.** Zur Herstellung der kosmetischen Zusammensetzung **A4** werden 9,3 g Emulsion **B1-a** mit 90,7 g Wasser verdünnt. Der Aktivgehalt an Organopolysiloxanen in der kosmetischen Zusammensetzung beträgt 3,0%.

**Vergleichsbeispiel VA4**

Kosmetische Zusammensetzung: Pflegendes Styling Spray

Das Vergleichsbeispiel **VA4** repräsentiert eine nicht-erfindungsgemäße kosmetische Zusammensetzung enthaltend die wässrige Dispersion **V2** eines vorvernetzten Organopolysiloxans. Bei der Herstellung der kosmetischen Zusammensetzung des Vergleichsversuches **VA4** wurde die Arbeitsweise des Beispiels **A4** wiederholt, mit der Abänderung, dass die vorvernetzte, nicht-erfindungsgemäße Emulsionen **V2** eingesetzt wird. 8,7 g Emulsion **V2** wurden mit 91,3 g Wasser verdünnt. Der Aktivgehalt an Organopolysiloxanen in der nicht erfindungsgemäßen kosmetischen Zusammensetzung **VA4** beträgt 3,0%.

**Vergleich der pflegenden Styling Sprays des erfindungsgemäßen Beispiels A4 mit dem Vergleichsversuch VA4**

Um die Formgebungseigenschaften der pflegenden Styling Sprays auf Haar zu untersuchen, wurden Haarlocken wie im Abschnitt "Curl Retention bei hoher Luftfeuchtigkeit" hergestellt, mit 0,2 g des jeweiligen Sprays eingesprüht und die Curl Retention ermittelt.

**Tabelle 13:** Pflegendes Styling Spray / Ergebnisse der Curl Retention bei hoher Luftfeuchtigkeit nach 7 h.

| Bsp. /Vgl. | Curl Retention [%] |
|---|---|
| Bsp. A4 | 73 |
| Vergleichsversuch VA4 | 30 |

Als Ergebnis erhielt man für Locken, die mit dem erfindungsgemäßen Styling Spray aus Beispiel **A4** behandelt wurden, eine Curl Retention von 73% nach 7 h. Die Locken fühlten sich angenehm weich an und erhielten ihre Form auch nach dem Kämmen. Das Kämmen der Locken war ohne Widerstand möglich. Für Haarlocken, behandelt mit dem Spray aus dem nicht-erfindungsgemäßen Vergleichsbeispiel **VA4** wurde eine Curl Retention von 30% ermittelt. Die Curl Retention bei hoher Luftfeuchtigkeit ist damit für diese Locken deutlich schlechter als bei Locken, die mit Spray aus dem erfindungsgemäßen Beispiel **A4** behandelt wurden.

**Patentansprüche**

1.  Wässrige Dispersionen, vorzugsweise wässrige Emulsionen, enthaltend vorvernetzte Organopolysiloxane,

    die durchschnittlich mindestens eine Struktureinheit der allgemeinen Formel

    $$SiRO_{2/2}\text{-}Y\text{-}SiRO_{2/2} \qquad (I)$$

    und Einheiten der Formel

    $$R_2SiO_{2/2} \qquad (II)$$

    enthalten, wobei
    Y ein Rest der Formel

    $$-R^2\text{-}[NR^3\text{-}R^4]_x\text{-}NR^3\text{-}OC\text{-}[C(Z^1)(H)]_{k1}\text{-}[C(Z^2)(H)]_{k2}\text{-}CO\text{-}NR^3$$
    $$-[R^4\text{-}NR^3]_a\text{-}R^2\text{-}R_2SiO_{1/2}\text{-}[R_2SiO_{2/2}]_b\text{-}R_2SiO_{1/2}\text{-}R^2\text{-}[NR^3\text{-}R^4]_a\text{-} \quad NR^3\text{-}OC\text{-}[C \quad (Z^2)(H)] \quad_{k2}\text{-}[C(Z^1)(H)]_{k1}\text{-}CO\text{-}$$
    $$NR^3\text{-}[R^4\text{-}NR^3]_x\text{-}R^2\text{-}$$

    bedeutet,
    R gleich oder verschieden sein kann und einen einwertigen SiC-gebundenen Kohlenwasserstoffrest mit 1 bis 18 Kohlenstoffatomen bedeutet, der ein oder mehrere Heteroatome aus der Gruppe N, P, S, O und Halogen enthalten kann,
    $R^2$ gleich oder verschieden sein kann und einen SiC-gebundenen, zweiwertigen linearen oder verzweigten Kohlenwasserstoffrest mit 3 bis 18 Kohlenstoffatomen, vorzugsweise einen Alkylenrest mit 3 bis 10 Kohlen-stoffatomen, bedeutet,
    $R^3$ gleich oder verschieden sein kann und ein Wasserstoffatom, einen Alkylrest mit 1 bis 8 Kohlenstoffatomen oder einen Acylrest, wie Acetylrest, bedeutet, vorzugsweise ein Wasserstoffatom ist,
    $R^4$ gleich oder verschieden sein kann und einen zweiwertigen Kohlenwasserstoffrest mit 1 bis 6 Kohlenstoffatomen, vorzugsweise einen Alkylenrest mit 1 bis 6 Kohlenstoffatomen, bedeutet,
    k1 0, 1, 2 oder 3 ist, vorzugsweise 0 ist,
    k2 0, 1, 2 oder 3 ist, vorzugsweise 0 ist,
    x 0, 1, 2, 3 oder 4, vorzugsweise 0 oder 1 ist,
    a 0, 1, 2, 3 oder 4, vorzugsweise 0 oder 1 ist,
    b 0 oder eine ganze Zahl von 1 bis 500, vorzugsweise 20 bis 350, ist,
    $Z^1$ -OH, H oder -NHR$^3$ bedeutet,
    $Z^2$ -OH, H oder -NHR$^3$ bedeutet.

2.  Wässrige Dispersionen nach Anspruch 1, **dadurch gekennzeichnet, dass** die vorvernetzten Organopolysiloxane Siloxaneinheiten der Formel

    $$R_{3\text{-}d}(OR^1)_dSiO_{1/2} \qquad (III)$$

enthalten, wobei

R die in Anspruch 1 dafür angegebene Bedeutung hat,

$R^1$ gleich oder verschieden sein kann und ein Wasserstoffatom oder einen Alkylrest mit 1 bis 18 Kohlenstoffatomen bedeutet, der durch ein oder mehrere separate Sauerstoffatome unterbrochen sein kann, und

d 0 oder 1 ist.

3. Wässrige Dispersionen nach Anspruch 1 oder 2, **dadurch gekennzeichnet, dass** Y ein Rest der Formel

$$-R^2-[NH-CH_2CH_2]_x-NH-OC-CO-NH-R^2 \qquad -R_2SiO_{1/2}-[R_2SiO_{2/2}]_b-R_2SiO_{1/2}- \qquad R^2-NH-OC-CO-$$
$$NH-[CH_2CH_2-NH]_x-R^2-$$

ist, wobei

R die in Anspruch 1 dafür angegebene Bedeutung hat,

x 0 oder 1 ist,

b 0 oder eine ganze Zahl von 1 bis 500, vorzugsweise 20 bis 350, ist und

$R^2$ ein Rest der Formel $-(CH_2)_3-$ oder $-CH_2-CH(CH_3)-CH_2-$ ist.

4. Verfahren zur Herstellung der wässrigen Dispersionen, vorzugsweise wässrigen Emulsionen, von vorvernetzten Organopolysiloxanen, indem
wässrige Dispersionen, vorzugsweise wässrige Emulsionen von Aminoorganopolysiloxanen (1) der Formel

$$(R^1O)_dA_eR_{3-d-e}SiO(SiARO)_p(SiR_2O)_qSiR_{3-d-e}A_e(OR^1)_d \qquad (IV)$$

wobei

A ein Aminorest der allgemeinen Formel

$$-R^2-[NR^3-R^4-]_xNR^3_2$$

ist,

R gleich oder verschieden sein kann und einen einwertigen SiC-gebundenen Kohlenwasserstoffrest mit 1 bis 18 Kohlenstoffatomen bedeutet, der ein oder mehrere Heteroatome aus der Gruppe N, P, S, O und Halogen enthalten kann,

$R^1$ gleich oder verschieden sein kann und ein Wasserstoffatom oder einen Alkylrest mit 1 bis 18 Kohlenstoffatomen bedeutet, der durch ein oder mehrere separate Sauerstoffatome unterbrochen sein kann,

$R^2$ gleich oder verschieden sein kann und einen SiC-gebundenen, zweiwertigen linearen oder verzweigten Kohlenwasserstoffrest mit 3 bis 18 Kohlenstoffatomen, vorzugsweise einen Alkylenrest mit 3 bis 10 Kohlenstoffatomen, bedeutet,

$R^3$ gleich oder verschieden sein kann und ein Wasserstoffatom, einen Alkylrest mit 1 bis 8 Kohlenstoffatomen oder einen Acylrest, wie Acetylrest, bedeutet, vorzugsweise ein Wasserstoffatom ist,

$R^4$ gleich oder verschieden sein kann und einen zweiwertigen Kohlenwasserstoffrest mit 1 bis 6 Kohlenstoffatomen, vorzugsweise einen Alkylenrest mit 1 bis 6 Kohlenstoffatomen, bedeutet,

d 0 oder 1 ist,

e 0 oder 1 ist,

p eine ganze Zahl von mindestens 1 und höchstens 1000 ist,

q 0 oder eine ganze Zahl von 1 bis 2000 ist, und

x 0, 1, 2, 3 oder 4, vorzugsweise 0 oder 1 ist,

mit reaktiven Estern (2) der Formel

$$R^5-O_2C-[C(Z^1)(H)]_{k1}-[C(Z^2)(H)]_{k2}-CO-NR^3$$

$$-[R^4-NR^3]_a-R^2-R_2SiO_{1/2}-[R_2SiO_{2/2}]_b-R_2SiO_{1/2}-R^2-[NR^3-R^4]_a- \qquad (V)$$

$$NR^3-OC-[C(Z^2)(H)]_{k2}-[C(Z^1)(H)]_{k1}-CO_2-R^5$$

wobei R, $R^2$, $R^3$ und $R^4$ die oben dafür angegebene Bedeutung haben,

$R^5$ gleich oder verschieden sein kann und einen O-gebundenen, gesättigten oder ungesättigten, linearen oder verzweigten, einwertigen Kohlenwasserstoffrest mit 1-20 Kohlenstoffatomen je Rest, der ein oder mehrere Heteroatome aus der Gruppe N, P, S, O und Halogen enthalten kann, bedeutet,

k1 0, 1, 2 oder 3 ist, vorzugsweise 0 ist,

k2 0, 1, 2 oder 3 ist, vorzugsweise 0 ist,

a 0, 1, 2, 3 oder 4, vorzugsweise 0 oder 1 ist,

b 0 oder eine ganze Zahl von 1 bis 500, vorzugsweise 20 bis 350, ist,

$Z^1$ -OH, H oder -NHR$^3$ bedeutet und

$Z^2$ -OH, H oder -NHR$^3$ bedeutet,

umgesetzt werden.

5. Verfahren nach Anspruch 4, **dadurch gekennzeichnet, dass** A ein Aminorest der Formel

$$-R^2-[NH-CH_2CH_2-]_xNH_2$$

ist, wobei

x 0 oder 1 ist und

$R^2$ ein Rest der Formel $-(CH_2)_3-$ oder $-CH_2-CH(CH_3)-CH_2-$ ist.

6. Verfahren nach Anspruch 4 oder 5, **dadurch gekennzeichnet, dass** die reaktiven Ester (2) Oxalamido-methylesterterminierte Organopolysiloxane und Oxalamido-ethylesterterminierte Organopolysiloxane sind.

7. Vorvernetzte Organopolysiloxane, die durchschnittlich mindestens eine Struktureinheit, vorzugsweise mindestens zwei Struktureinheiten, bevorzugt mindestens drei Struktureinheiten, der allgemeinen Formel

$$SiRO_{2/2}-Y-SiRO_{2/2} \qquad (I)$$

und Einheiten der Formel

$$R_2SiO_{2/2} \qquad (II)$$

enthalten, wobei

Y ein Rest der Formel

$$-R^2-[NR^3-R^4]_x-NR^3OC-[C(Z^1)(H)]_{k1}-[C(Z^2)(H)]_{k2}-CO-NR^3-[R^4-NR^3]_a-R^2-R_2SiO_{1/2}-[R_2SiO_{2/2}]_b-R_2SiO_{1/2}-R^2-[NR^3-R^4]_a- \qquad NR^3-OC-[C(Z^2)(H)]_{k2}-[C(Z^1)(H)]_{k1}-CO-NR^3-[R^4-NR^3]_x-R^2-$$

bedeutet,

R, $R^2$, $R^3$, $R^4$, k1, k2, x, a, b, $Z^1$ und $Z^2$ die in Anspruch 1 dafür angegebene Bedeutung haben.

8. Vorvernetzte Organopolysiloxane nach Anspruch 7, **dadurch gekennzeichnet, dass** sie Siloxaneinheiten der Formel

$$R_{3-d}(OR^1)_dSiO_{1/2} \qquad (III)$$

enthalten, wobei

R die in Anspruch 1 dafür angegebene Bedeutung hat und

d und $R^1$ die in Anspruch 2 dafür angegebene Bedeutung haben.

9. Verfahren zur Herstellung der vorvernetzten Organopolysiloxane, **dadurch gekennzeichnet, dass** Aminoorganopolysiloxane der Formel

$$(R^1O)_dA_eR_{3-d-e}SiO(SiARO)_p(SiR_2O)_qSiR_{3-d-e}A_e(OR^1)_d \qquad (IV)$$

mit reaktiven Estern der Formel

$$R^5\text{-}O_2C\text{-}[C(Z^1)(H)]_{k1}\text{-}[C(Z^2)(H)]_{k2}\text{-}CO\text{-}NR^3 \quad - \quad [R^4\text{-}NR^3]_a\text{-}R^2\text{-}R_2SiO_{1/2}\text{-}[R_2SiO_{2/2}]_b\text{-}R_2SiO_{1/2}\text{-}R^2\text{-}[NR^3R^4]_a\text{-} \quad (V)$$

$NR^3\text{-}OC\text{-}[C(Z^2)(H)]_{k2}\text{-}[C(Z^1)(H)]_{k1}\text{-}CO_2\text{-}R^5$ wobei

A, R, $R^1$, $R^2$, $R^3$, $R^4$, $R^5$, d, e, p, q, k1, k2, a, b, $Z^1$ und $Z^2$ die in Anspruch 4 dafür angegebene Bedeutungen haben, umgesetzt werden,
und die so erhaltenen vorvernetzten Organopolysiloxane gegebenenfalls anschließend in Wasser emulgiert werden.

10. Kosmetische Zusammensetzungen enthaltend wässrige Dispersionen, vorzugsweise wässrige Emulsionen, von vorvernetzten Organopolysiloxanen nach Anspruch 1, 2 oder 3 oder hergestellt nach Anspruch 4, 5 oder 6 oder vorvernetzte Organopolysiloxane nach Anspruch 7 oder 8 oder hergestellt nach Anspruch 9.

11. Kosmetische Zusammensetzungen nach Anspruch 10, **dadurch gekennzeichnet, dass** sie Konditionierungsmittel enthalten.

12. Verwendung der kosmetischen Zusammensetzungen nach Anspruch 10 oder 11 zur Behandlung von keratinischen Fasern, vorzugsweise zur Reinigung, Pflege oder Formgebung von keratinischen Fasern.

13. Verwendung der kosmetischen Zusammensetzungen nach Anspruch 10 oder 11 zum Konditionieren von keratinischen Fasern, insbesondere um die Kämmbarkeit von keratinischen Fasern zu erleichtern.

14. Verwendung nach Anspruch 12 oder 13, **dadurch gekennzeichnet, dass** die keratinischen Fasern Haare sind.

15. Verfahren zur Behandlung von keratinhaltigen Fasern, vorzugsweise Haaren, indem die kosmetischen Zusammensetzungen nach Anspruch 10 oder 11 auf die keratinischen Fasern, vorzugsweise Haare, aufgetragen werden und dann gegebenenfalls mit Wasser gespült werden.

## Claims

1. Aqueous dispersions, preferably aqueous emulsions, comprising precrosslinked organopolysiloxanes that contain on average at least one structural unit of the general formula

$$SiRO_{2/2}\text{-}Y\text{-}SiRO_{2/2} \quad (I)$$

and units of the formula

$$R_2SiO_{2/2} \quad (II)$$

where

Y denotes a radical of the formula

$$-R^2\text{-}[NR^3\text{-}R^4]_x\text{-}NR^3\text{-}OC\text{-}[C(Z^1)(H)]_{k1}\text{-}[C(Z^2)(H)]_{k2}\text{-}CO\text{-}NR^3 \quad - \quad [R^4\text{-}NR^3]_a\text{-}R^2\text{-}R_2SiO_{1/2}\text{-}[R_2SiO_{2/2}]_b\text{-}R_2SiO_{1/2}\text{-}R^2\text{-}[NR^3\text{-}R^4]_a\text{-} \quad NR^3\text{-}OC\text{-}[C \quad (Z^2)(H)]_{k2}\text{-}[C(Z^1)(H)]_{k1}\text{-}CO\text{-}NR^3\text{-}[R^4\text{-}NR^3]_x\text{-}R^2\text{-},$$

R may be identical or different and denotes a monovalent SiC-bonded hydrocarbon radical that has 1 to 18 carbon atoms and may contain one or more heteroatoms from the group of N, P, S, O, and halogen,
$R^2$ may be identical or different and denotes a SiC-bonded, divalent linear or branched hydrocarbon radical having 3 to 18 carbon atoms, preferably an alkylene radical having 3 to 10 carbon atoms,
$R^3$ may be identical or different and denotes a hydrogen atom, an alkyl radical having 1 to 8 carbon atoms or an acyl radical, such as acetyl radical, and preferably is a hydrogen atom,
$R^4$ may be identical or different and denotes a divalent hydrocarbon radical having 1 to 6 carbon atoms, preferably an alkylene radical having 1 to 6 carbon atoms,
k1 is 0, 1, 2 or 3, preferably 0,
k2 is 0, 1, 2 or 3, preferably 0,
x is 0, 1, 2, 3 or 4, preferably 0 or 1,
a is 0, 1, 2, 3 or 4, preferably 0 or 1,
b is 0 or an integer from 1 to 500, preferably 20 to 350,
$Z^1$ denotes -OH, H or -$NHR^3$,
$Z^2$ denotes -OH, H or -$NHR^3$.

2. Aqueous dispersions according to Claim 1, **characterized in that** the precrosslinked organopolysiloxanes contain siloxane units of the formula

$$R_{3-d}(OR^1)_d SiO_{1/2} \qquad (III)$$

where

R is as defined in Claim 1,
$R^1$ may be identical or different and denotes a hydrogen atom or an alkyl radical that has 1 to 18 carbon atoms and may be interrupted by one or more separate oxygen atoms, and
d is 0 or 1.

3. Aqueous dispersions according to Claim 1 or 2, **characterized in that** Y is a radical of the formula

$$-R^2-[NH-CH_2CH_2]_x-NH-OC-CO-NH-R^2 \qquad -R_2SiO_{1/2}-[R_2SiO_{2/2}]_b-R_2SiO_{1/2}- \qquad R^2-NH-OC-CO-NH-[CH_2CH_2-NH]_x-R^2-$$

where

R is as defined in Claim 1,
x is 0 or 1,
b is 0 or an integer from 1 to 500, preferably 20 to 350, and
$R^2$ is a radical of the formula -$(CH_2)_3$- or -$CH_2$-$CH(CH_3)$-$CH_2$-.

4. Process for producing aqueous dispersions, preferably aqueous emulsions, of precrosslinked organopolysiloxanes, wherein aqueous dispersions, preferably aqueous emulsions, of aminoorganopolysiloxanes (1) of the formula

$$(R^1O)_d A_e R_{3-d-e} SiO(SiARO)_p (SiR_2O)_q SiR_{3-d-e} A_e(OR^1)_d \qquad (IV)$$

where

A is an amino radical of the general formula

$$-R^2-[NR^3-R^4-]_x NR^3_2,$$

R may be identical or different and denotes a monovalent SiC-bonded hydrocarbon radical that has 1 to 18 carbon atoms and may contain one or more heteroatoms from the group of N, P, S, O, and halogen,
$R^1$ may be identical or different and denotes a hydrogen atom or an alkyl radical that has 1 to 18 carbon atoms and may be interrupted by one or more separate oxygen atoms,
$R^2$ may be identical or different and denotes a SiC-bonded, divalent linear or branched hydrocarbon radical having 3 to 18 carbon atoms, preferably an alkylene radical having 3 to 10 carbon atoms,
$R^3$ may be identical or different and denotes a hydrogen atom, an alkyl radical having 1 to 8 carbon atoms or an acyl radical, such as acetyl radical, and preferably is a hydrogen atom,
$R^4$ may be identical or different and denotes a divalent hydrocarbon radical having 1 to 6 carbon atoms, preferably an alkylene radical having 1 to 6 carbon atoms,
d is 0 or 1,
e is 0 or 1,
p is an integer that is at least 1 and at most 1000,
q is 0 or an integer from 1 to 2000, and

x is 0, 1, 2, 3 or 4, preferably 0 or 1,
are reacted with reactive esters (2) of the formula

$$R^5\text{-}O_2C\text{-}[C(Z^1)(H)]_{k1}\text{-}[C(Z^2)(H)]_{k2}\text{-}CO\text{-}NR^3$$
$$[R^4\text{-}NR^3]_a\text{-}R^2\text{-}R_2SiO_{1/2}\text{-}[R_2SiO_{2/2}]_b\text{-}R_2SiO_{1/2}\text{-}R^2\text{-}[NR^3\text{-} \qquad (V)$$

$$R^4]_a\text{-}NR^3\text{-}OC\text{-}[C(Z^2)(H)]_{k2}\text{-}[C(Z^1)(H)]_{k1}\text{-}CO_2\text{-}R^5$$
where R, $R^2$, $R^3$ and $R^4$ are as defined above,
$R^5$ may be identical or different and denotes an O-bonded, saturated or unsaturated, linear or branched, monovalent hydrocarbon radical that has 1-20 carbon atoms per radical and may contain one or more heteroatoms from the group of N, P, S, O and halogen,
k1 is 0, 1, 2 or 3, preferably 0,
k2 is 0, 1, 2 or 3, preferably 0,
a is 0, 1, 2, 3 or 4, preferably 0 or 1,
b is 0 or an integer from 1 to 500, preferably 20 to 350,
$Z^1$ denotes -OH, H or -NHR$^3$, and
$Z^2$ denotes -OH, H or -NHR$^3$.

5. Process according to Claim 4, **characterized in that** A is an amino radical of the formula

$$-R^2\text{-}[NH\text{-}CH_2CH_2\text{-}]_xNH_2$$

where

x is 0 or 1 and
$R^2$ is a radical of the formula $-(CH_2)_3-$ or $-CH_2\text{-}CH(CH_3)\text{-}CH_2-$.

6. Process according to Claim 4 or 5, **characterized in that** the reactive esters (2) are oxalamido-methyl-ester-terminated organopolysiloxanes and oxalamido-ethylester-terminated organopolysiloxanes.

7. Precrosslinked organopolysiloxanes that contain on average at least one structural unit, preferably at least two structural units, more preferably at least three structural units, of the general formula

$$SiRO_{2/2}\text{-}Y\text{-}SiRO_{2/2} \qquad (I)$$

and units of the formula

$$R_2SiO_{2/2} \qquad (II)$$

where

Y denotes a radical of the formula

$$-R^2\text{-}[NR^3\text{-}R^4]_x\text{-}NR^3\text{-}OC\text{-}[C(Z^1)(H)]_{k1}\text{-}[C(Z^2)(H)]_{k2}\text{-}CO\text{-}NR^3$$
$$[R^4\text{-}NR^3]_a\text{-}R^2\text{-}R_2SiO_{1/2}\text{-}[R_2SiO_{2/2}]_b\text{-}R_2SiO_{1/2}\text{-}R^2\text{-}[NR^3\text{-}R^4]_a\text{-} \qquad NR^3\text{-}OC\text{-}[C(Z^2)(H)]_{k2}\text{-}[C(Z^1)(H)]_{k1}\text{-}CO\text{-}$$
$$NR^3\text{-}[R^4\text{-}NR^3]_x\text{-}R^2\text{-},$$
R, $R^2$, $R^3$, $R^4$, k1, k2, x, a, b, $Z^1$ and $Z^2$ are as defined in Claim 1.

8. Precrosslinked organopolysiloxanes according to Claim 7, **characterized in that** they contain siloxane units of the formula

$$R_{3-d}(OR^1)_dSiO_{1/2} \qquad (III)$$

where

R is as defined in Claim 1 and
d and $R^1$ are as defined in Claim 2.

9. Process for producing precrosslinked organopolysiloxanes, **characterized in that** aminoorganopolysiloxanes of the formula

$$(R^1O)_d A_e R_{3-d-e} SiO(SiARO)_p (SiR_2O)_q SiR_{3-d-e} A_e (OR^1)_d \qquad (IV)$$

are reacted with reactive esters of the formula

$$R^5\text{-}O_2C\text{-}[C(Z^1)(H)]_{k1}\text{-}[C(Z^2)(H)]_{k2}\text{-}CO\text{-}NR^3$$

$$\text{-}[R^4\text{-}NR^3]_a\text{-}R^2\text{-}R_2SiO_{1/2}\text{-}[R_2SiO_{2/2}]_b\text{-}R_2SiO_{1/2}\text{-}R^2\text{-}[NR^3\text{-} \qquad (V)$$

$$R^4]_a\text{-}NR^3\text{-}OC\text{-}[C(Z^2)(H)]_{k2}\text{-}[C(Z^1)(H)]_{k1}\text{-}CO_2\text{-}R^5$$

where

A, R, $R^1$, $R^2$, $R^3$, $R^4$, $R^5$, d, e, p, q, k1, k2, a, b, $Z^1$ and $Z^2$ are as defined in Claim 4,
and the resulting precrosslinked organopolysiloxanes are optionally subsequently emulsified in water.

10. Cosmetic compositions comprising
aqueous dispersions, preferably aqueous emulsions, of precrosslinked organopolysiloxanes according to Claim 1, 2 or 3 or produced according to Claim 4, 5 or 6, or precrosslinked organopolysiloxanes according to Claim 7 or 8 or produced according to Claim 9.

11. Cosmetic compositions according to Claim 10, **characterized in that** they comprise conditioning agents.

12. Use of the cosmetic compositions according to Claim 10 or 11 for treating keratin fibers, preferably for cleansing, caring for or shaping keratin fibers.

13. Use of the cosmetic compositions according to Claim 10 or 11 for conditioning keratin fibers, for the purpose in particular of making keratin fibers easier to comb.

14. Use according to Claim 12 or 13, **characterized in that** the keratin fibers are hair.

15. Process for treating keratin-containing fibers, preferably hair, by applying the cosmetic compositions according to Claim 10 or 11 to the keratin fibers, preferably hair, and then optionally rinsing with water.

**Revendications**

1. Dispersions aqueuses, de préférence émulsions aqueuses, contenant des organopolysiloxanes préréticulés, qui contiennent en moyenne au moins un motif structural de formule générale

$$SiRO_{2/2}\text{-}Y\text{-}SiRO_{2/2} \qquad (I)$$

et des motifs de formule

$$R_2SiO_{2/2} \qquad (II)$$

dans lesquelles

Y signifie radical de formule

$$\text{-}R^2\text{-}[NR^3\text{-}R^4]_x\text{-}NR^3\text{-}OC\text{-}[C(Z^1)(H)]_{k1}\text{-}[C(Z^2)(H)]_{k2}\text{-}CO\text{-}NR^3 \qquad \text{-}$$
$$[R^4\text{-}NR^3]_a\text{-}R^2\text{-}R_2SiO_{1/2}\text{-}[R_2SiO_{2/2}]_e\text{-}R_2SiO_{1/2}\text{-}R^2\text{-}[NR^3\text{-}R^4]_a\text{-} \qquad NR^3\text{-}OC\text{-}[C(Z^2)(H)]_{k2}\text{-}[C(Z^1)(H)]_{k1}\text{-}CO\text{-}$$
$$NR^3\text{-}[R^4\text{-}NR^3]_x\text{-}R^2\text{-},$$

R peut être identique ou différent et signifie un radical hydrocarboné monovalent lié par SiC comprenant 1 à 18 atomes de carbone, qui peut contenir un ou plusieurs hétéroatomes du groupe constitué par N, P, S, O et

halogène,

$R^2$ peut être identique ou différent et signifie un radical hydrocarboné divalent, linéaire ou ramifié, lié par SiC, comprenant 3 à 18 atomes de carbone, de préférence un radical alkylène comprenant 3 à 10 atomes de carbone,

$R^3$ peut être identique ou différent et signifie un atome d'hydrogène, un radical alkyle comprenant 1 à 8 atomes de carbone ou un radical acyle, tel qu'un radical acétyle, de préférence un atome d'hydrogène,

$R^4$ peut être identique ou différent et signifie un radical hydrocarboné divalent comprenant 1 à 6 atomes de carbone, de préférence un radical alkylène comprenant 1 à 6 atomes de carbone,

k1 vaut 0, 1, 2 ou 3, de préférence 0,

k2 vaut 0, 1, 2 ou 3, de préférence 0,

x vaut 0, 1, 2, 3 ou 4, de préférence 0 ou 1,

a vaut 0, 1, 2, 3 ou 4, de préférence 0 ou 1,

b vaut 0 ou un nombre entier de 1 à 500, de préférence de 20 à 350,

$Z^1$ signifie -OH, H ou -NHR$^3$,

$Z^2$ signifie -OH, H ou -NHR$^3$.

2.  Dispersions aqueuses selon la revendication 1, **caractérisées en ce que** les organopolysiloxanes préréticulés contiennent des motifs siloxane de formule

$$R_{3-d}(OR^1)_d SiO_{1/2} \qquad (III)$$

dans laquelle

R présente la signification indiquée dans la revendication 1 pour ce radical,

$R^1$ peut être identique ou différent et signifie un atome d'hydrogène ou un radical alkyle comprenant 1 à 18 atomes de carbone, qui peut être interrompu par un ou plusieurs atomes d'oxygène séparés et

d vaut 0 ou 1.

3.  Dispersions aqueuses selon la revendication 1 ou 2, **caractérisées en ce que** Y représente un radical de formule

$$-R^2-[NH-CH_2CH_2]_x-NH-OC-CO-NH-R^2 \qquad -R_2SiO_{1/2}-[R_2SiO_{2/2}]_b-R_2SiO_{1/2}- \qquad R^2-NH-OC-CO-NH-[CH_2CH_2-NH]_x-R^2-$$

dans laquelle

R présente la signification indiquée dans la revendication 1 pour ce radical,

x vaut 0 ou 1,

b vaut 0 ou un nombre entier de 1 à 500, de préférence de 20 à 350 et

$R^2$ représente un radical de formule - $(CH_2)_3$- ou -CH$_2$-CH(CH$_3$)-CH$_2$-.

4.  Procédé de préparation des dispersions aqueuses, de préférence des émulsions aqueuses, d'organopolysiloxanes préréticulés dans lequel

des dispersions aqueuses, de préférence des émulsions aqueuses, d'aminoorganopolysiloxanes (1) de formule

$$(R^1O)_d A_e R_{3-d-e} SiO(SiARO)_p (SiR_2O)_q SiR_{3-d-e} A_e (OR^1)_d \qquad (IV)$$

dans laquelle

A représente un radical amino de formule générale

$$-R^2-[NR^3-R^4-]_x NR^3_2,$$

R peut être identique ou différent et signifie un radical hydrocarboné monovalent lié par SiC comprenant 1 à 18 atomes de carbone, qui peut contenir un ou plusieurs hétéroatomes du groupe constitué par N, P, S, O et halogène,

$R^1$ peut être identique ou différent et signifie un atome d'hydrogène ou un radical alkyle comprenant 1 à 18 atomes de carbone, qui peut être interrompu par un ou plusieurs atomes d'oxygène séparés,

$R^2$ peut être identique ou différent et signifie un radical hydrocarboné divalent, linéaire ou ramifié, lié par SiC, comprenant 3 à 18 atomes de carbone, de préférence un radical alkylène comprenant 3 à 10 atomes de carbone,

$R^3$ peut être identique ou différent et signifie un atome d'hydrogène, un radical alkyle comprenant 1 à 8 atomes de carbone ou un radical acyle, tel qu'un radical acétyle, de préférence un atome d'hydrogène,

$R^4$ peut être identique ou différent et signifie un radical hydrocarboné divalent comprenant 1 à 6 atomes de carbone, de préférence un radical alkylène comprenant 1 à 6 atomes de carbone,

d vaut 0 ou 1,

e vaut 0 ou 1,

p vaut un nombre entier d'au moins 1 et d'au plus 1000,

q vaut 0 ou un nombre entier de 1 à 2000 et

x vaut 0, 1, 2, 3 ou 4, de préférence 0 ou 1,

sont transformées avec des esters réactifs (2) de formule

$$R^5-O_2C-[C(Z^1)(H)]_{k1}-[C(Z^2)(H)]_{k2}-CO-NR^3$$

$$- [R^4-NR^3]_a-R^2-R_2SiO_{1/2}-[R_2SiO_{2/2}]_e-R_2SiO_{1/2}-R^2-[NR^3- \qquad (V)$$

$$R^4]_a- NR^3-OC-[C(Z^2)(H)]_{k2}-[C(Z^1)(H)]_{k1}-CO_2-R^5$$

dans laquelle R, $R^2$, $R^3$ et $R^4$ présentent la significations indiquées ci-dessus pour ces radicaux,

$R^5$ peut être identique ou différent et signifie un radical hydrocarboné monovalent, saturé ou insaturé, linéaire ou ramifié, lié par O, comprenant 1 à 20 atomes de carbone par radical, qui peut contenir un ou plusieurs hétéroatomes du groupe constitué par N, P, S, O et halogène,

k1 vaut 0, 1, 2 ou 3, de préférence 0,

k2 vaut 0, 1, 2 ou 3, de préférence 0,

a vaut 0, 1, 2, 3 ou 4, de préférence 0 ou 1,

b vaut 0 ou un nombre entier de 1 à 500, de préférence de 20 à 350,

$Z^1$ signifie -OH, H ou -NHR$^3$ et

$Z^2$ signifie -OH, H ou -NHR$^3$.

5. Procédé selon la revendication 4, **caractérisé en ce que** A représente un radical amino de formule

$$-R^2-[NH-CH_2CH_2-]_xNH_2$$

dans laquelle

x vaut 0 ou 1 et

$R^2$ représente un radical de formule $-(CH_2)_3-$ ou $-CH_2-CH(CH_3)-CH_2-$.

6. Procédé selon la revendication 4 ou 5, **caractérisé en ce que** les esters réactifs (2) sont des organopolysiloxanes terminés par ester oxalamidométhylique et des organopolysiloxanes terminés par ester oxalamidoéthylique.

7. Organopolysiloxanes préréticulés qui contiennent en moyenne au moins un motif structural, de préférence au moins deux motifs structuraux, de préférence au moins trois motifs structuraux, de formule générale

$$SiRO_{2/2}-Y-SiRO_{2/2} \qquad (I)$$

et des motifs de formule

$$R_2SiO_{2/2} \qquad (II)$$

dans lesquelles

Y signifie radical de formule

$$-R^2-[NR^3-R^4]_x-NR^3-OC-[C(Z^1)(H)]_{k1}-[C(Z^2)(H)]_{k2}-CO-NR^3$$
$$-[R^4-NR^3]_a-R^2-R_2SiO_{1/2}-[R_2SiO_{2/2}]_b-R_2SiO_{1/2}-R^2-[NR^3-R^4]_a- \qquad NR^3-OC-[C(Z^2)(H)]_{k2}-[C(Z^1)(H)]_{k1}-CO-$$
$$NR^3-[R^4-NR^3]_x-R^2-,$$

R, $R^2$, $R^3$, $R^4$, k1, k2, x, a, b, $Z^1$ et $Z^2$ présentent la signification indiquée dans la revendication 1 pour ces éléments.

8. Organopolysiloxanes préréticulés selon la revendication 7, **caractérisés en ce qu'**ils contiennent des motifs siloxane de formule

$$R_{3-d}(OR^1)_d SiO_{1/2} \qquad (III)$$

dans laquelle

R présente la signification indiquée dans la revendication 1 pour ce radical et
d et $R^1$ présentent la signification indiquée dans la revendication 2 pour ces éléments.

9. Procédé de préparation des organopolysiloxanes préréticulés, **caractérisé en ce que** des aminoorganopolysiloxanes de formule

$$(R^1O)_d A_e R_{3-d-e} SiO(SiARO)_p (SiR_2O)_q SiR_{3-d-e} A_e (OR^1)_d \qquad (IV)$$

sont transformés avec des esters réactifs de formule

$$R^5-O_2C-[C(Z^1)(H)]_{k1}-[C(Z^2)(H)]_{k2}-CO-NR^3$$
$$-[R^4-NR^3]_a-R^2-R^2SiO_{1/2}-[R_2SiO_{2/2}]_b-R^2SiO_{1/2}-R^2-[NR^3- \qquad (V)$$
$$R^4]_a-NR^3-OC-[C(Z^2)(H)]_{k2}-[C(Z^1)(H)]_{k1}-CO_2-R^5 \text{ dans lesquelles}$$

A, R, $R^1$, $R^2$, $R^3$, $R^4$, $R^5$, d, e, p, q, k1, k2, a, b, $Z^1$ et $Z^2$ ont les significations indiquées dans la revendication 4 pour ces éléments,
et les organopolysiloxanes préréticulés ainsi obtenus sont éventuellement ensuite émulsionnés dans l'eau.

10. Compositions cosmétiques contenant
des dispersions aqueuses, de préférence des émulsions aqueuses, d'organopolysiloxanes préréticulés selon la revendication 1, 2 ou 3 ou préparées selon la revendication 4, 5 ou 6 ou des organopolysiloxanes préréticulés selon la revendication 7 ou 8 ou préparés selon la revendication 9.

11. Compositions cosmétiques selon la revendication 10, **caractérisées en ce qu'**elles contiennent des agents conditionneurs.

12. Utilisation des compositions cosmétiques selon la revendication 10 ou 11 pour le traitement de fibres kératiniques, de préférence pour le nettoyage, le soin ou la mise en forme de fibres kératiniques.

13. Utilisation des compositions cosmétiques selon la revendication 10 ou 11 pour le conditionnement de fibres kératiniques, en particulier pour améliorer l'aptitude au peignage de fibres kératiniques.

14. Utilisation selon la revendication 12 ou 13, **caractérisée en ce que** les fibres kératiniques sont des cheveux.

15. Procédé de traitement de fibres kératiniques, de préférence de cheveux, dans lequel les compositions cosmétiques selon la revendication 10 ou 11 sont appliquées sur les fibres kératiniques, de préférence des cheveux, puis éventuellement rincées à l'eau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2019114953 A1
- US 7501184 B2
- US 7223385 B2
- US 7485289 B2
- US 7220408 B2
- US 7504094 B2
- WO 2020239229 A1
- US 5039738 A
- WO 2015024079 A1
- WO 2004039930 A2
- US 5399652 A
- US 7129369 B2
- WO 20239229 A1

### In der Beschreibung aufgeführte Nicht-Patentliteratur

- **M.D. BERTHIAUME**. Society of Cosmetic Chemists (Hrsg.. *Monograph, Silicones in Hair Care*, 1997
- **J. SEJPKA**. Silicone in Haarpflegeprodukten. *SÖFW-Journal*, 1992, vol. 118 (17), 1065-1070
- Wetting Properties of Human Hair by Means of Dynamic Contact Angle Measurement. **R.A. LODGE** ; **B. BHUSAN**. Journal of Applied Polymer Science. Wiley, 2006, vol. 102, 5255-5265
- **K. KRUMMEL** ; **STEPHANE CHIRON** ; **J. JACHOWICZ**. *The Chemistry and Manufacture of Cosmetics*, 2000, vol. II (14), 359-396
- *CHEMICAL ABSTRACTS*, 1309-48-4
- *CHEMICAL ABSTRACTS*, 70131-67-8
- *CHEMICAL ABSTRACTS*, 497-19-8
- **Y. K. KAMATH** ; **HANS-DIETRICH WEIGMANN**. *J. Soc. Cosmet. Chem.*, 1986, vol. 37, 111-124